# EUROPEAN PATENT APPLICATION

(11) **EP 2 606 907 A1**
(43) Date of publication of application: **26.06.2013**
(21) Application number: 11306702.9
(22) Date of filing: 19.12.2011
(51) Int. Cl.: A61K 38/17, C07K 14/435

(54) **Optineurin-derived polypeptides, their nucleic acids and uses thereof**

(71) Applicant: Institut Pasteur, 75724 Paris Cédex 15 (FR); Centre National de la Recherche Scientifique, 75794 Paris Cedex 16 (FR); UNIVERSITE PIERRE ET MARIE CURIE (PARIS 6), 75005 Paris (FR)
(72) Inventor: Weil, Robert, Serge, 92210 Saint-Cloud (FR); Kachaner, David, 75003 Paris (FR); Côrte-Real Filipe, Josina, 2710-195 Sintra (PT); Laplantine, Emmanuel, François, 93400 Saint-Ouen (FR); Israël, Alain, 75014 Paris (FR)
(74) Representative: Desaix, Anne

(57) **Abstract**

The invention relates to Optineurin-derived polypeptide(s) consisting of a polypeptidic sequence disclosed in SEQ ID N°2, which represents the portion of the wild-type human Optineurin protein sequence from its amino-acid residue 131 to its amino-acid residue 297, or having a polypeptidic sequence encompassing the polypeptidic sequence disclosed in SEQ ID N°2, or having a polypeptidic sequence derived from the polypeptidic sequence disclosed in SEQ ID N°2 to the exclusion of the wild-type Optineurin protein. The polypeptide(s) of the invention retain one, any combination of two, or three of the following functional properties: the capacity to bind Rab8 protein, when the latter are associated with the Golgi apparatus of a cell, the capacity to bind MYPT1 protein, when said protein is engaged in a Myosin-Phosphatase (MP) complex, the capacity to be phosphorylated by Plk1. The invention also relates to nucleic acid molecule(s) encoding such polypeptide(s), vector(s) and method(s) of production thereof, as well as uses thereof, in particular uses in treating cancer(s) or in treating a disease involving mitosis deregulation(s) or alteration(s).

SEQUENCE LISTING

<110>  INSTITUT PASTEUR
       CENTRE NATIONAL DE LA RECHERCHE SCIENTIFIQUE
       UNIVERSITE PIERRE ET MARIE CURIE

<120>  OPTINEURIN-DERIVED POLYPEPTIDES, THEIR NUCLEIC ACIDS AND USES
       THEREOF

<130>  B09670 AD/AFL

<140>  EP XXXX
<141>  2011-12-19

<160>  10

<170>  PatentIn version 3.5

<210>  1
<211>  577
<212>  PRT
<213>  homo sapiens

<220>
<221>  MISC_FEATURE
<222>  (1)..(577)
<223>  optineurin protein sequence - NCBI accession number
       NP_001008214.1

<400>  1

<210>  2
<211>  167
<212>  PRT
<213>  homo sapiens

<220>
<221>  MISC_FEATURE
<222>  (1)..(167)
<223>  optineurin protein fragment between residues 131 and 297

<400>  2

Leu Pro Arg Ala Glu Ala Glu Gln Glu Lys Asp Gln Leu Arg Thr Gln
1               5                   10                  15

Val Val Arg Leu Gln Ala Glu Lys Ala Asp Leu Leu Gly Ile Val Ser
            20                  25                  30

Glu Leu Gln Leu Lys Leu Asn Ser Ser Gly Ser Ser Glu Asp Ser Phe
        35                  40                  45

Val Glu Ile Arg Met Ala Glu Gly Glu Ala Glu Gly Ser Val Lys Glu
    50                  55                  60

Ile Lys His Ser Pro Gly Pro Thr Arg Thr Val Ser Thr Gly Thr Ala
65                  70                  75                  80

Leu Ser Lys Tyr Arg Ser Arg Ser Ala Asp Gly Ala Lys Asn Tyr Phe
                85                  90                  95

Glu His Glu Glu Leu Thr Val Ser Gln Leu Leu Leu Cys Leu Arg Glu
            100                 105                 110

Gly Asn Gln Lys Val Glu Arg Leu Glu Val Ala Leu Lys Glu Ala Lys
        115                 120                 125

Glu Arg Val Ser Asp Phe Glu Lys Lys Thr Ser Asn Arg Ser Glu Ile
    130                 135                 140

Glu Thr Gln Thr Glu Gly Ser Thr Glu Lys Glu Asn Asp Glu Glu Lys
145                 150                 155                 160

Gly Pro Glu Thr Val Gly Ser
                165

<210>  3
<211>  167
<212>  PRT
<213>  Artificial Sequence

<220>
<223>  optineurin protein fragment (131-297) mutated at positions 151,
       157 and 177

<400>  3

Leu Pro Arg Ala Glu Ala Glu Gln Glu Lys Asp Gln Leu Arg Thr Gln
1               5                   10                  15

Val Val Arg Pro Gln Ala Glu Lys Ala Asp Pro Leu Gly Ile Val Ser
            20                  25                  30

Glu Leu Gln Leu Lys Leu Asn Ser Ser Gly Ser Ser Glu Asp Ala Phe
        35                  40                  45

Val Glu Ile Arg Met Ala Glu Gly Glu Ala Glu Gly Ser Val Lys Glu
    50                  55                  60

Ile Lys His Ser Pro Gly Pro Thr Arg Thr Val Ser Thr Gly Thr Ala
65                  70                  75                  80

Leu Ser Lys Tyr Arg Ser Arg Ser Ala Asp Gly Ala Lys Asn Tyr Phe
                85                  90                  95

Glu His Glu Glu Leu Thr Val Ser Gln Leu Leu Leu Cys Leu Arg Glu
            100                 105                 110

Gly Asn Gln Lys Val Glu Arg Leu Glu Val Ala Leu Lys Glu Ala Lys
        115                 120                 125

Glu Arg Val Ser Asp Phe Glu Lys Lys Thr Ser Asn Arg Ser Glu Ile
    130                 135                 140

Glu Thr Gln Thr Glu Gly Ser Thr Glu Lys Glu Asn Asp Glu Glu Lys
145                 150                 155                 160

Gly Pro Glu Thr Val Gly Ser
                165

<210>  4
<211>  15
<212>  PRT
<213>  Homo sapiens

<220>
<221>  MISC_FEATURE
<222>  (1)..(15)
<223>  plk1 phosphorylation sequence (Figure 1B)

<400>  4

Ser Ser Gly Ser Ser Glu Asp Ser Phe Val Glu Ile Arg Met Ala
1               5                   10                  15

<210>  5
<211>  15
<212>  PRT
<213>  Mus musculus

<220>
<221>  MISC_FEATURE
<222>  (1)..(15)
<223>  plk1 phosphorylation sequence (Figure 1B)

<400>  5

Ser Gly Gly Ser Ser Glu Asp Ser Phe Val Glu Ile Arg Met Thr
1               5                   10                  15

<210> 6
<211> 15
<212> PRT
<213> Sus scrofa

<220>
<221> MISC_FEATURE
<222> (1)..(15)
<223> plk1 phosphorylation sequence (Figure 1B)

<400> 6

Ser Gly Gly Pro Ser Glu Asp Ser Phe Val Glu Ile Arg Met Ala
1               5                   10                  15

<210> 7
<211> 15
<212> PRT
<213> Gallus gallus

<220>
<221> MISC_FEATURE
<222> (1)..(15)
<223> plk1 phosphorylation sequence (Figure 1B)

<400> 7

Asn Ile Ala Ser Ala Glu Asp Ser Phe Val Glu Ile Gly Met Asn
1               5                   10                  15

<210> 8
<211> 15
<212> PRT
<213> Xenopus laevis

<220>
<221> MISC_FEATURE
<222> (1)..(15)
<223> plk1 phosphorylation sequence (Figure 1B)

<400> 8

Leu Gly Ser Phe Ser Glu Asp Ser Phe Val Glu Ile Gly Phe Ser
1               5                   10                  15

<210> 9
<211> 1734
<212> DNA
<213> homo sapiens

<220>
<221> misc_feature
<222> (1)..(1734)
<223> optineurin coding DNA

<400> 9
atgtcccatc aacctctcag ctgcctcact gaaaaggagg acagccccag tgaaagcaca

ggaaatggac ccccccacct ggcccaccca aacctggaca cgtttacccc ggaggagctg

ctgcagcaga tgaaagagct cctgaccgag aaccaccagc tgaaagaagc catgaagcta

aataatcaag ccatgaaagg gagatttgag gagctttcgg cctggacaga gaaacagaag

gaagaacgcc agttttttga gatacagagc aaagaagcaa aagagcgtct aatggccttg

agtcatgaga atgagaaatt gaaggaagag cttggaaaac taaaagggaa atcagaaagg

tcatctgagg accccactga tgactccagg cttcccaggg ccgaagcgga gcaggaaaag

gaccagctca ggacccaggt ggtgaggcta caagcagaga aggcagacct gttgggcatc

gtgtctgaac tgcagctcaa gctgaactcc agcggctcct cagaagattc ctttgttgaa

attaggatgg ctgaaggaga agcagaaggg tcagtaaaag aaatcaagca tagtcctggg

cccacgagaa cagtctccac tggcacggca ttgtctaaat ataggagcag atctgcagat

ggggccaaga attacttcga acatgaggag ttaactgtga gccagctcct gctgtgccta

agggaaggga atcagaaggt ggagagactt gaagttgcac tcaaggaggc caaagaaaga

gtttcagatt ttgaaaagaa aacaagtaat cgttctgaga ttgaaaccca gacagagggg

agcacagaga aagagaatga tgaagagaaa ggcccggaga ctgttggaag cgaagtggaa

gcactgaacc tccaggtgac atctctgttt aaggagcttc aagaggctca tacaaaactc

agcaaagctg agctaatgaa gaagagactt caagaaaagt gtcaggccct tgaaaggaaa

aattctgcaa ttccatcaga gttgaatgaa aagcaagagc ttgtttatac taacaaaaag

ttagagctac aagtggaaag catgctatca gaaatcaaaa tggaacaggc taaaacagag

gatgaaaagt ccaaattaac tgtgctacag atgacacaca acaagcttct tcaagaacat

aataatgcat tgaaaacaat tgaggaacta acaagaaaag agtcagaaaa agtggacagg

gcagtgctga aggaactgag tgaaaaactg gaactggcag agaaggctct ggcttccaaa

cagctgcaaa tggatgaaat gaagcaaacc attgccaagc aggaagagga cctggaaacc

atgaccatcc tcagggctca gatggaagtt tactgttctg attttcatgc tgaaagagca

gcgagagaga aaattcatga ggaaaaggag caactggcat tgcagctggc agttctgctg

aaagagaatg atgctttcga agacggaggc aggcagtcct tgatggagat gcagagtcgt

catggggcga gaacaagtga ctctgaccag caggcttacc ttgttcaaag aggagctgag

gacagggact ggcggcaaca gcggaatatt ccgattcatt cctgccccaa gtgtggagag

gttctgcctg acatagacac gttacagatt cacgtgatgg attgcatcat ttaa

<210> 10
<211> 167
<212> PRT
<213> Artificial Sequence

<220>
<223> Optineurin protein segment 131-297 mutated at position 177

<400>  10

Leu Pro Arg Ala Glu Ala Glu Gln Glu Lys Asp Gln Leu Arg Thr Gln
1               5                   10                  15

Val Val Arg Leu Gln Ala Glu Lys Ala Asp Leu Leu Gly Ile Val Ser
            20                  25                  30

Glu Leu Gln Leu Lys Leu Asn Ser Ser Gly Ser Ser Glu Asp Ala Phe
        35                  40                  45

Val Glu Ile Arg Met Ala Glu Gly Glu Ala Glu Gly Ser Val Lys Glu
    50                  55                  60

Ile Lys His Ser Pro Gly Pro Thr Arg Thr Val Ser Thr Gly Thr Ala
65                  70                  75                  80

Leu Ser Lys Tyr Arg Ser Arg Ser Ala Asp Gly Ala Lys Asn Tyr Phe
                85                  90                  95

Glu His Glu Glu Leu Thr Val Ser Gln Leu Leu Leu Cys Leu Arg Glu
            100                 105                 110

Gly Asn Gln Lys Val Glu Arg Leu Glu Val Ala Leu Lys Glu Ala Lys
        115                 120                 125

Glu Arg Val Ser Asp Phe Glu Lys Lys Thr Ser Asn Arg Ser Glu Ile
    130                 135                 140

Glu Thr Gln Thr Glu Gly Ser Thr Glu Lys Glu Asn Asp Glu Glu Lys
145                 150                 155                 160

Gly Pro Glu Thr Val Gly Ser
                165

## Description

The invention is directed to polypeptidic sequence fragments of human Optineurin or mutated Optineurin protein and mutated fragments of Optineurin. The compounds of the invention have properties showing their interest for the purpose of interfering with the cascade of events involved in the mitotic process.

The invention also relates to compositions, antibodies, nucleic acid molecules, vectors or cells necessary for achieving such a purpose, as well as methods of therapy or *in vitro* methods using them.

Defects within the mitotic process are involved in severe diseases, such as cancer(s). Therefore, there is a tremendous need for understanding the pathways involved in mitotic progression of a cell as well as a need to design tools for efficiently dealing with defective mitotic progression. Acting at the molecular level, tools may be designed for compensating a deficient function leading to a defective mitotic progression or tools may be designed for taking advantage of a defective process in a defective cell, such as in a cancer cell, with a view to effectively restore a molecular equilibrium promoting mitotic progression and completion within a cell or to favour cell death of cells undergoing abnormal mitosis. Understanding pathways involved in the mitotic process allowed the inventors to design products achieving fine-tuning of the subtly balanced system that is mitosis. More generally, this understanding might be useful in the design of laboratory tools aimed at further exploring the complex and dynamic interactions that can be found at the cellular level, in particular during mitotic progression.

The invention relies on experiments performed by the inventors that allowed concluding that PIk1-dependent phosphorylation of Optineurin provides a negative feedback mechanism for mitotic progression.

The ability of cells to divide is essentially attributed to cyclin-dependent kinases (Cdks) and their substrates (Satyanarayana and Kaldis, 2009). In addition to Cdk1, other mitotic kinases such as polo-like kinases, orthologues of fly Aurora, or NIMA-related kinases play essential roles in mitotic checkpoints, mitotic exit and cytokinesis (Salaun et al., 2008).

Polo-like kinases belong to a conserved subfamily of serine/threonine protein kinases that play pivotal roles in the cell cycle progression (Archambault and Glover, 2009). Plk1 contributes to several critical events of cell division including G2/M transition, centrosome maturation, bipolar spindle formation, chromosome segregation, activation of the anaphase-promoting complex/cyclosome (APC/C) and exit from mitosis with the initiation of cytokinesis (Archambault and Glover, 2009). It is thought that these PIk1 activities are linked to its subcellular localization. In prometaphase and metaphase, PIk1 is targeted to the centromeric and kinetochore regions of chromosomes where it might be involved for the stable attachment of kinetochores to microtubules (Archambault and Glover, 2009). At the metaphase-anaphase transition, PIk1 relocalized to the spindle midzone, where it acts as a key regulator of cytokinesis. Defects in cytokinesis frequently cause formation of bi-nucleate or polyploid cells (Glotzer, 2005). Consistent with these roles, downregulation of PIk1 activity results in mitotic arrest and cell death in mammalian cells (Liu and Erikson, 2003; Sumara et al., 2004). PIk1 is composed of an amino-terminus catalytic domain and a carboxy-terminus Polo Box Domain (PBD) that acts as a phosphopeptide-binding domain and targets PIk1 to its subcellular locations by binding proteins that have been previously phosphorylated by "priming" kinases such as Cdk1 (Elia et al., 2003). The activity of PIk1 is also temporally regulated: PIk1 expression increases through interphase progression and peaks during the G2 phase in correlation with its kinase activity that reaches its a maximum at the G2/M transition. This temporal control is achieved by transcriptional regulation, phosphorylation and proteolysis. PIk1 is activated at mitotic entry by phosphorylation in the T-loop on threonine 210 by the synergistic action of Bora and the kinase Aurora A (Macurek et al., 2008; Seki et al., 2008). In contrast, the myosin phosphatase complex MYPT1/PP1β counteracts the phosphorylation of PIk1 on Thr210 (Yamashiro et al., 2008).

Optineurin ("optic neuropathy inducing", Optn) is a multifunctional protein expressed in human cells and that has been shown to be linked to different human pathologies. Mutations within the *Optn* gene have been associated with familial forms of primary open-angle glaucoma, Amyotrophic lateral sclerosis and genetic variants Optn are also a genetic risk factor for Paget's disease (Albagha et al., 2010; Chung et al., 2010; Maruyama et al., 2010; Rezaie et al., 2002).

The inventors designed and performed experiments proving that phosphorylation of Optn on Ser177 by PIk1 impairs its association with the small GTPase Rab8 and induces its translocation into the nucleus during mitosis. To identify interacting proteins that could potentially account for a role of Optn during mitosis, a biochemical/proteomic approach was performed. The PIk1 inhibitory phosphatase complex MYPT1-PP1β (MP) was identified. The term PP1cβ can sometimes be found in the literature instead of PP1β, both are strictly equivalents. Phosphorylation of MYPT1 by Cdk1 is required for interaction with PIk1 PBD, thereby allowing dephosphorylation of PIk1 by PP1β (Yamashiro et al., 2008). Importantly, the inventors observed that Optn functionally connects MYPT1 and PP1β to PIk1 during mitosis by promoting phosphorylation of MYPT1. It was then found that Optn depletion increases PIk1 phosphorylation (at Thr210) and kinase activity, resulting in multinucleation and cytokinetic defects. These phenotypes were rescued upon expression of siRNA-insensitive wild type Optn, but not when the phosphodeficient mutant S177A was expressed, which mutant cannot translocate into the nucleus during mitosis. In addition, the inventors found that silencing Optn could rescue PIk1 mitotic effects, such as recruitment of γ-tubulin to the mitotic centrosomes, that was impaired by the partial knockdown of PIk1. Overall, these results highlight the role of Optn in the regulation of PIk1 during mitosis, and further reveal a feedback mechanism by which PIk1 modulates Optn localization in order to regulate its own activity.

In view of the identified interactions taking place during mitosis cascade events in human cells, the inventors provided compounds, especially polypeptides, that proved to be suitable for interfering with mitosis completion, especially impairing mitosis completion, through interaction with PIk1 activity regulation.

Therefore, the invention relates to polypeptide(s) that is/are capable to interfer with the Optineurin-mediated PIk1 auto-regulation loop in a cell.

Within the context of the invention, a "cell" is a prokaryotic or eukaryotic cell, particularly an animal cell, and more particularly a mammalian cell such as human cell(s) or non-human mammalian cell(s). In a particular embodiment for *in vitro* applications of the invention, cells, before transformation, are isolated from either a primary culture or cell line(s).

Cells used within the context of the invention are dividing cells. By "dividing cell", it is meant a cell that is capable to enter or has entered mitosis. Cells can be issued from a primary culture, meaning a culture prepared from cells or tissues directly from an animal or a plant, or from cell line(s), encompassing a population of cells resulting from a primary or first culture.

The cellular interacting partners for Optineurin that were identified in the newly discovered Optineurin-mediated PIk1 auto-regulation loop are either Rab8 protein present on the Golgi apparatus or MYPT1 protein, including MYPT1 protein engaged in a Myosin-Phosphatase (MP) complex when associated with PP1β. By definition, within the context of the present disclosure, a Myosin-Phosphatase (MP) complex involves MYPT1 and PP1β proteins.

This enabled the inventors to design polypeptides of the invention taking into account the particular features of the discovered pathway, and to provide polypeptides interfering with the binding of endogenous Optineurin with at least one of its partner(s) in mitotic cells. Expected results are for example increasing the availability of endogenous Optineurin for further PIk1 phosphorylation or lowering said availability. Phosphorylation of Optineurin protein(s) by PIk1 itself was found to be an entry point into the newly discovered pathway, since this phosphorylation correlates in time with the dissociation between Optineurin protein(s) and the Rab8 protein(s) of the Golgi apparatus at mitosis onset.

By "endogenous Optineurin" or "wild-type Optineurin", it is meant the Optineurin protein that can be naturally found in a cell, or that is naturally produced by the genome of said cell. In other words endogeneous or wild type Optineurin is the protein expressed in a cell naturally containing a gene encoding Optineurin, and whose genotype has not been modified deliberately with a polynucleotide encoding Optineurin or whose phenotype has not been deliberately modified as a result of contacting the cell with Optineurin.

Wild-type Optineurin is especially human wild-type Optineurin, whose amino-acid sequence can for example be retrieved from Genbank database, for example under NCBI references NP_001008214.1 and corresponds, according to a preferred embodiment, to a polypeptide having an amino-acid sequence of SEQ ID N°1.

The invention therefore relates to a polypeptide consisting of polypeptidic sequence disclosed in SEQ ID N°2. Alternatively, it relates to a polypeptide having a polypeptidic sequence encompassing the polypeptidic sequence disclosed in SEQ ID N°2 which represents the portion of the wild-type human Optineurin protein sequence from its amino-acid residue 131 to its amino-acid residue 297, or having a polypeptidic sequence derived from the polypeptidic sequence disclosed in SEQ ID N°2 to the exclusion of the wild-type Optineurin protein.

In particular, such a derived polyeptide has a polypeptidic sequence with functional identity in all or some of the functional properties of the polypeptidic sequence disclosed in SEQ ID N°2 which represents the portion of the wild-type human Optineurin protein sequence from its amino-acid residue 131 to its amino-acid residue 297.

Within the context of the present invention, a polypeptidic sequence having "functional identity" with the polypeptidic sequence disclosed in SEQ ID N°2 retains one, any combination of two, or three of the functional properties of said sequence, choosen amongst: the capacity to bind Rab8 protein, when the latter are associated with the Golgi apparatus of a cell, the capacity to bind MYPT1 protein, when said protein is engaged in a Myosin-Phosphatase (MP) complex, the capacity to be phosphorylated by PIk1.

In a particular embodiment, a polypeptide of the invention is mutated with respect to SEQ ID N°2, or derives from SEQ ID N°2 so as to bear only one or two of the functional properties described above.

In a particular embodiment, a polypeptide of the invention has a polypeptidic sequence encompassing the polypeptidic sequence disclosed in SEQ ID N°2 or a polypeptidic sequence derived from the polypeptidic sequence disclosed in SEQ ID N°2, especially mutated with respect to said SEQ ID N°2.

In a specific embodiment, a derived polypeptide of the invention has an amino acid sequence derived from the polypeptidic sequence disclosed in SEQ ID N°2 and has an amino acid sequence which shares at least 75%, or 80%, 85%, 90%, 95% or 99% identity over its whole length with the sequence set forth in SEQ ID N°2.

By "derived from" it is specified that the polypeptide has a structure, especially a secondary amino-acids structure in common with the polypeptide having sequence of SEQ ID N°2. It may however be obtained from any convenient source, having recourse to any appropriate method.

By "identity", it is meant that the percentage of conserved amino-acid residues when a mutant polypeptide of the invention is aligned with SEQ ID N°2 through conventional alignment algorithms is substantial, meaning that this percentage is at least 75%.

By "identity over its whole length", it is meant that the identity between a polypeptide of the invention and SEQ ID N°2 is calculated through a global alignment algorithm taking as reference the sequence of the polypeptide of SEQ ID N°2. An example of such a global alignment algorithm is the Needleman and Wunsch algorithm. Gaps are allowed when such an alignment is performed.

According to a particular embodiment, a polypeptide of the invention has a minimal length of roughly 40, 49 or 50 amino-acid residues, or 87, 88, 89 or 90 amino-acid residues. Possible lengths for a polypeptide of the invention however encompass 120, 125, 130, 135, 140, 145, 150, 155, 160, 165 or 166 amino-acids, up to roughly 207 or 240 amino-acids. For example, a polypeptide consisting of a fragment of the wild-type human Optineurin protein sequence from its amino-acid residue 58 to its amino-acid residue 297, which fragments binds Rab8 or/and MYPT1 protein, and possesses a PIk1 phosphorylation sequence comprising amino-acid residues at position 177 by reference to the Optineurin protein sequence SEQ ID N°1.

According to a particular embodiment, such a polypeptide of the invention has a length of roughly 49 amino-acid residues and comprises a domain binding MYPT1 and possesses a PIk1 phosphorylation sequence, in particular consists of amino-acid residues 131 to 180 in the sequence of SEQ ID N°1.

According to a particular embodiment, such a polypeptide of the invention has a length of roughly 39 amino-acid residues and comprises a domain binding MYPT1 and possesses a PIk1 phosphorylation sequence, in particular consists of amino-acid residues 141 to 180 in the sequence of SEQ ID N°1.

According to a particular embodiment, such a polypeptide of the invention has a length of roughly 78 amino-acid residues, binds Rab8 and comprises a domain binding MYPT1, and possesses a PIk1 phosphorylation sequence, in particular consists of amino-acid residues 131 to 209 in the sequence of SEQ ID N°1.

According to a particular embodiment, such a polypeptide of the invention has a length of roughly 68 amino-acid residues, binds Rab8 and comprises a domain binding MYPT1, and possesses a PIk1 phosphorylation sequence, in particular consists of amino-acid residues 141 to 209 in the sequence of SEQ ID N°1.

According to a particular embodiment, such a polypeptide of the invention has a length of roughly 88 amino-acid residues, binds Rab8 and comprises a domain binding MYPT1, and possesses a PIk1 phosphorylation sequence, in particular consists of amino-acid residues 131 to 219 in the sequence of SEQ ID N°1.

According to a particular embodiment, such a polypeptide of the invention has a length of roughly 78 amino-acid residues, binds Rab8 and comprises a domain binding MYPT1, and possesses a PIk1 phosphorylation sequence, in particular consists of amino-acid residues 141 to 219 in the sequence of SEQ ID N°1.

According to a particular embodiment, a polypeptide of the invention has a length of roughly 120 to 166 amino-acid residues, and has at least 75% identity with SEQ ID N°2 when they are aligned over the whole length of SEQ ID N°2 through conventional alignment algorithms.

According to particular embodiments, a polypeptide of the invention is "derived" or "mutated" as to retain only one or a combination of two of the functional properties referred to above. Said polypeptides have an amino-acid sequence which shares at least 75%, or 80%, 85%, 90%, 95% or 99% identity over its whole length with the sequence set forth in SEQ ID N°1 or SEQ ID N°2.

According to a particular embodiment, a polypeptide of the invention that is non-mutated with respect to known or reference wild-type Optineurin(s) sequences, is a fragment of said known wild-type or reference Optineurin(s) sequences, meaning that such a polypeptide is necessarily shorter than the corresponding known wild-type Optineurin sequence.

In another embodiment of the invention, a polypeptide is a fragment of wild-type Optineurin which is longer than the fragment having SEQ ID N° 2.

In a particular embodiment, a polypeptide of the invention has a sequence that is non-mutated with respect to a continuous fragment of the wild-type human Optineurin protein, especially a continuous fragment of at least 49 amino-acids with respect to said wild-type human Optineurin protein.

For the purpose of the present disclosure, when the polypeptidic sequence of a polypeptide of the invention differs from the polypeptidic sequence SEQ ID N° 2 referred to here-above, said polypeptide of the invention is defined as a derived or mutated polypeptide. The modification(s) defining the mutated polypeptide(s) of the invention can independently be deletion(s), including especially point deletion(s) of one or many amino acid residue(s) or can be substitution(s), especially conservative substitution(s) of one or many amino acid residue(s).

Such conservative substitutions encompass a change of residues made in consideration of specific properties of amino acid residues as disclosed in the following groups of amino acid residues and the resulting substituted polypeptide should not be modified functionally:
Acidic: Asp, Glu;
Basic: Asn, Gln, His, Lys, Arg;
Aromatic: Trp, Tyr, Phe;
Uncharged Polar Side chains: Asn, Gly, Gln, Cys, Ser, Thr, Tyr;
Nonpolar Side chains: Ala, Val, Leu, Ileu, Pro, Phe, Met, Trp ;
Hydrophobic: Ile, Val, Leu, Phe, Cys, Met, Nor;
Neutral Hydrophilic: Cys, Ser, Thr ;
Residues impacting chain orientation: Gly, Pro
Small amino acid residues: Gly, Ala, Ser.

In another embodiment, depending on the property(ies) guiding the choice for substitution of amino acid residue(s), modification of residue(s) can alternatively be determined to modify the properties of the resulting polypeptide, and said substitution(s) are selected to be non conservative.

Furthermore, phosphorylation of Optineurin protein(s) at amino-acid residue 177 by PIk1 itself was found as an interesting feature for the design of polypeptide of the invention, since this event was found to be an entry point into the discovered negative regulation loop of PIk1 activity.

According to a particular embodiment, a polypeptide of the invention has a polypeptide sequence that comprises a PIk1 phosphorylation consensus sequence D/E-X-S-φ-X-D/E (X being any amino acid residue; φ being a hydrophobic amino acid residue). Accordingly, such a polypeptidic sequence enables a polypeptide of the invention to be phosphorylated by PIk1. For example, such a PIk1 phosphorylation consensus sequence comprises amino-acid residue(s) at position 177 by reference to the Optineurin protein sequence SEQ ID N°1.

In specific embodiment(s), e.g. when the amino-acid sequence of the polypeptide of the invention consists of the sequence SEQ ID N°2, the PIk1 phosphorylation consensus sequence comprises amino-acid residues at position 177 by reference to the Optineurin protein sequence SEQ ID N°1.

According to particular specific embodiments, such polypeptides of the invention, i.e. polypeptides having PIk1 phosphorylation consensus sequences, have polypeptidic sequences that comprise any one of the polypeptidic sequences disclosed under SEQ ID N°4 to SEQ ID N°8 or a fragment of at least 70 amino-acid residues of SEQ ID N° 2, which comprises the PIk1 phosphorylation sequence including amino-acid residues at position 177 by reference to the sequence of SEQ ID N° 1.

According to a particular embodiment, a polypeptide of the invention:
i. is mutated in the PIk1 phosphorylation sequence in a way that it does not enable phosphorylation of said polypeptide, especially by PIk1, in particular is mutated at position 177 by reference to the Optineurin protein sequence SEQ ID N°1 so as to prevent phosphorylation of the amino-acid residue at said position 177, in particular has an Alanine residue at said position 177, and,
ii. optionally does not have the capacity of the wild-type human Optineurin protein to bind MYPT1 protein, when said MYPT1 protein is engaged in a Myosin-Phosphatase (MP) complex.

According to a particular embodiment, when a polypeptide of the invention discloses a PIk1 phosphorylation consensus sequence, it also retains the capacity of the wild-type Optineurin protein to bind MYPT1 protein, when said MYPT1 protein is engaged in a Myosin-Phosphatase (MP) complex.

According to the present invention, a "Myosin-Phosphatase (MP) complex" encompasses a complex resulting from the association between MYPT1 and PP1β as functional partners. According to a particular embodiment, the presence of both MYPT1 and PP1β associated in a functional entity is required for said entity to connect Optineurin protein(s) during mitosis, thereby allowing phosphorylation of MYPT1 and its translocation into the nucleus of a cell under a complex resulting from the association of MP and phosphorylated Optineurin, and allowing interaction with the Cdk1 protein present in the nucleus, leading to inactivation of PIk1.

The expression "to bind" means that a tight association between the bound elements, by way of physical or functional interactions.

According to a particular embodiment, the binding of a polypeptide of the invention with MYPT1 protein correlates with the presence of a Leucine Zipper (LZ) domain on the polypeptidic sequence of a polypeptide of the invention. In particular, said binding can be achieved through interaction of MYPT1 protein with such a Leucine Zipper (LZ) domain.

According to a particular embodiment, a polypeptide of the invention comprises a Leucine Zipper (LZ) domain that is upstream from its PIk1 phosphorylation consensus sequence. When meaningful, said Leucine Zipper (LZ) domain is in particular located upstream from residue corresponding to position 177 by reference to the Optineurin protein sequence SEQ ID N°1.

According to a particular embodiment, a polypeptide of the invention has the amino-acid sequence SEQ ID N°2.

In contrast with the embodiment wherein a polypeptide of the invention comprises a PIk1 phosphorylation consensus sequence, a polypeptide of the invention can alternatively, according to another particular embodiment, comprise a mutated PIk1 phosphorylation consensus sequence that does not enable phosphorylation of said polypeptide, especially by PIk1. In such a particular embodiment, a polypeptide of the invention is considered to be a nonphosphorylatable mutant.

Phospho-mimicking polypeptide mutants are excluded from this embodiment.

According to a specific embodiment, a polypeptide of the invention is mutated at position 177 by reference to the Optineurin protein sequence SEQ ID N°1, or at a position corresponding to or matching said position 177 in the case of a variant polypeptide with respect to SEQ ID N°1, so as to be a nonphosphorylatable mutant.

Within the context of the invention, when a mutation at position 177 or a similar position is present in a polypeptide of the invention, said mutation is aimed at preventing phosphorylation of the amino-acid residue at said position. More specifically with respect to this embodiment, it is observed that an Alanine residue instead of a Serine residue at said position 177, by reference to SEQ ID N°1, prevents phosphorylation of said residue by PIk1. Such a result is achieved with a polypeptidic sequence such as SEQ ID N°3 or SEQ ID N°10.

According to a specific embodiment, the PIk1 phosphorylation consensus sequence of a mutated polypeptide of the invention presents a S177A substitution.

According to a particular embodiment, when a polypeptide of the invention is considered to be a nonphosphorylatable mutant, such a polypeptide is nevertheless not functionally impaired with respect to its capacity to bind MYPT1 protein, when said MYPT1 protein is engaged in a Myosin-Phosphatase (MP) complex.

According to a particular embodiment, when a polypeptide of the invention is considered to be a nonphosphorylatable mutant, such a polypeptide is also functionally impaired with respect to its capacity to bind MYPT1 protein, when said MYPT1 protein is engaged in a Myosin-Phosphatase (MP) complex.

According to a particular embodiment, the MYPT1 protein(s)-binding property of a polypeptide of the invention are conferred by the presence of a Leucine Zipper (LZ) domain within the polypeptidic sequence of a polypeptide of the invention. According to a particular embodiment, such a Leucine Zipper (LZ) domain is found upstream of a PIk1 phosphorylation consensus sequence, which is mutated or not, in said polypeptide, and in particular is the polypeptide segment having amino-acid residues 151 to 157 defined by reference to SEQ ID N° 1.

Accordingly, the polypeptidic sequence of a polypeptide of the invention is mutated in a portion encompassing a Leucine Zipper (LZ) domain located upstream from the mutated PIk1 phosphorylation consensus sequence of said polypeptide, so as to disrupt the function of said Leucine Zipper (LZ) domain, or is devoid of such a Leucine Zipper (LZ) domain, so as to disrupt the function of said Leucine Zipper (LZ) domain. It can be expected from such a mutation that it prevents a polypeptide of the invention from interacting with MYPT1 protein, including MYPT1 protein engaged in a Myosin-Phosphatase (MP) complex.

According to a particular embodiment, a polypeptide of the invention can be mutated both in its PIk1 phosphorylation consensus sequence and within a LZ domain so as it would be expected from such a polypeptide not to be enabled to be phosphorylated by PIk1 and not to interact with MYPT1 protein.

According to a particular embodiment, a polypeptide of the invention has the amino-acid sequence SEQ ID N°3 or is a fragment of this polypeptide comprising the mutated sites for phosphorylation and LZ.

According to a particular embodiment, mutation(s) within a LZ domain of a polypeptide of the invention do(es) not affect the capability of said polypeptide to interact with Rab8 protein.

Additionally, the embodiment excluding both the presence of PIk1 phosphorylation site and the presence of a domain capable of interacting with MYPT1 protein, such as a Leucine Zipper (LZ) domain, allows for the preparation of polypeptides of the invention of shorter length, with respect to the length of roughly 120 amino-acid residues mentioned here-above.

In a particular embodiment, a polypeptide of the invention encompasses or consists of the wild-type human Optineurin protein sequence from its amino-acid residue 131 to its amino-acid residue 219, said polypeptide being mutated in its Leucine Zipper (LZ) domain, for example at positions 151 and/or 157 so as to disrupt said LZ domain function, and bearing a S177A mutation. Such a polypeptide is 88 amino-acids long and interacts with Rab8 protein.

According to specific embodiments, the polypeptides referred to above can also be mutated so as to disrupt one or more of the functional properties of the SEQ ID N°2, as previously disclosed.

In a particular embodiment, a polypeptide of the invention has additional amino-acid residue(s), such as a Cysteine residue, at its N-terminal or C-terminal extremity(ies). The presence of a free Cysteine residue may be of interest to enable attachment of additional moieties, especially markers or tags or other active groups.

However, according to another particular embodiment, no specific amino-acid residue is required at the N-terminal or C-terminal extremity(ies) of a polypeptide of the invention to achieve attachment of additional moieties, since any amino-acid carboxy group or another chemical group of a polypeptide of the invention can be used to this end.

In a particular embodiment of the invention, a polypeptide of the invention comprises or is constituted by L-amino acid residues.

In a particular embodiment, a polypeptide of the invention comprises or is fully constituted by D-amino acids (excluding the chiral form of amino acids naturally synthesized by living organisms, which is the L-form), or comprises or is fully constituted by modified aminoacids. Such modifications might help preventing proteolytic cleavage by active enzymes, especially when the polypeptide is administered *in vivo.*

According to a particular embodiment, polypeptide(s) of the invention is/are labelled, especially by coupling with a fluorophore such as Cy5, Cys5.5, or a biotin, or result(s) from a fusion protein aimed at grafting a fluorescent moiety to polypeptide(s) of the invention, such as a GFP fragment. According to a particular embodiment, polypeptide(s) of the invention is/are coupled with moiety(ies) aimed at facilitating its/their separation or isolation, such as GST fragment. Coupling can also be achieved through the production of a fusion protein, merging a polypeptide of the invention with moiety(ies) of interest in a single molecule.

The polypeptides of the invention encompass polypeptides and peptidomimetic molecules that can be prepared by conventional routes, in particular that can be chemically synthesized, for example through Solid-Phase synthesis, or engineered through biotechnological methods.

When polypeptide(s) of the invention are prepared in recombinant cells, these cells are recombined with a polynucleotide expressing the polypeptide, using nucleic acid expression systems such as plasmid vectors, which include, as an insert, a polynucleotide encoding a polypeptide of the invention.

The invention therefore also relates to nucleic acid molecule(s) encoding polypeptidic sequence(s) of polypeptide(s) of the invention as described herein and nucleic acid expression system(s), especially vector(s), such as cloning vector(s) or expression vector(s) comprising such nucleic acid molecule(s).

Said vector(s) can include expression control sequences or permit the production of fusion polypeptide(s) or fusion protein(s).

The invention also relates to nucleic acid molecule(s) encoding a polypeptide, in particular a mutated polypeptide, of the invention. Said nucleic acid molecule(s) are prepared by conventional techniques as either a single stand molecule or a double strand molecule of complementary sequences. They are especially DNA or cDNA molecules

In a particular embodiment, a nucleic acid molecule of the invention consists of a fragment of the nucleic acid sequence set forth in SEQ ID N°9 or of a fragment of the nucleic acid sequence that is complementary to SEQ ID N°9.

According to a particular embodiment, the sequence of a nucleic acid molecule of the invention is mutated with respect to the sequence set forth above, in order to correspond to the mutated polypeptide(s) of the invention as defined above.

According to a particular embodiment, production of isolated polypeptide(s) of the invention is achieved through the transfection of such vector(s) in cell(s) such as E.coli cell(s) or eukaryotic cells, including yeast cells, insect cells or mammalian cells, especially human cells, the culture of said cell(s) and the recovery of the protein result of the culture, especially the recovery of the purified polypeptide(s) of the invention.

The invention thus also relates to a vector, especially a plasmid, comprising a nucleic acid molecule as defined herein.

The invention also relates to method for producing a polypeptide of the invention comprising the transfection of a cell with a vector as described herein, the culture of said cell and the recovery of said polypeptide.

The invention also encompasses cell(s) or population of cells comprising a nucleic acid molecule or a vector as described herein, especially for use in a method of production of isolated or purified polypeptide(s) of the invention.

According to another particular embodiment, a polypeptide of the invention is produced through chemical synthesis.

It is another object of the invention to impact the regulation of PIk1 activity in a dividing cell through the discovered Optineurin-mediated PIk1 auto-regulation loop pathway, by either increasing or decreasing the negative regulation of PIk1 activity by Optineurin in a cell, thereby impairing or therapeutically interfering with the cascade of events involved in said cell mitosis. As a consequence, when contacted with a cell, a polypeptide of the invention modulates the subcellular distribution between the cytoplasm and the nucleus of the endogenous Optineurin protein of said cell.

Mitosis events that can be impaired as a result can be G2/M transition, centrosome maturation, bipolar spindle formation, chromosome segregation, activation of the anaphase-promoting complex/cyclosome (APC/C) and exit from mitosis with the initiation of cytokinesis (Archambault and Glover, 2009).

The invention therefore also relates to polypeptide(s) for use either to enhance or to counteract negative regulation of PIk1 activity by Optineurin in a dividing cell, thereby impairing or therapeutically interfering with the cascade of events involved in said cell when undergoing mitosis, wherein said polypeptide binds in said cell to at least one cellular partner for Optineurin selected amongst Rab8 protein and MYPT1 protein, including when said MYPT1 protein is engaged in a Myosin-Phosphatase (MP) complex, and thereby modulates the subcellular distribution of the endogenous Optineurin protein between the cytoplasm and the nucleus in said cell.

As discussed above, the fact that a polypeptide can bind at least one cellular partner for Optineurin in a cell chosen amongst Rab8 protein and MYPT1 protein means that such a polypeptide can interact with the newly discovered Optineurin-mediated PIk1 auto-regulation loop in a dividing cell.

As a result, by providing a mitotic cell with a polypeptide, an use according to the invention further seeks the impairment, i.e. blocking, of the cascade of events involved in the mitosis of a cell, or seeks a therapeutic influence on said cascade of events.

By "therapeutic influence" it is meant that the use of a polypeptide as disclosed herein in a dividing cell results in an observable modification of the phenotype of said cell, which is beneficial to the condition, or health state of an animal or human patient to whom it is administered or might lead to excess of mitosis cycles (such as in tumor cells) that favour their proliferation.

PIk1 activity can be naturally regulated in a normally functioning cell, for example through the newly discovered Optineurin-mediated PIk1 auto-regulation loop. Such a regulation of PIk1 activity might not be present in a dysfunctional cell. However, by "enhancing or counteracting negative regulation of PIk1 activity in a cell", or "increasing or decreasing the negative regulation of PIk1 activity in a cell", it is meant that the invention seeks a modification, a change or a modulation of the activity of PIk1 protein(s) within said cell during mitosis, independently of either the presence or the absence of a normal PIk1 activity regulation function in said cell. Accordingly, the terms "enhance" ("increase") and "counteract" ("decrease") used above are defined with respect to the mechanism(s) present in a normally regulated cell for PIk1 negative regulation by endogenous Optineurin. By "normally regulated cell" it is meant a cell that is not subject to any deleterious condition resulting from either an external factor or an internal factor for PIk1 negative regulation by endogenous Optineurin.

By "enhancing (increasing) the negative regulation of PIk1 activity", it is expected to decrease PIk1 activity. Within the context of the present invention, this action encompasses the fact of suppressing said PIk1 activity in a cell.

By "counteracting (decreasing) the negative regulation of PIk1 activity", it is expected to increase PIk1 activity.

Knowing that PIk1 can be found under an activated (phosphorylated) form with phosphorylation of amino-acid residue 210 and under an inactivated (dephosphorylated) form, modulation of regulation of PIk1 activity in a cell can be achieved, for example, by promoting PIk1 activation or by promoting PIk1 inactivation. The phosphorylation status of the Threonin amino-acid residue at position 210 by reference to PIk1 polypeptidic sequence thus defines whether PIk1 is under its activated or inactivated form, i.e., a phosphorylated Threonin amino-acid residue defines the PIk1 activated form, and a dephosphorylated Threonin amino-acid residue defines the PIk1 inactivated form.

Since PIk1 is involved in several critical stages of the mitotic process, i.e. in G2/M transition, centrosome maturation, bipolar spindle formation, chromosome segregation, activation of the anaphase-promoting complex/cyclosome (APC/C) and exit from mitosis with the initiation of cytokinesis (Archambault and Glover, 2009), acting on the regulation of PIk1 activity, especially through the discovered Optineurin-mediated PIk1 auto-regulation loop pathway, necessarily results in interfering in the completion of mitosis at various stages, e.g. by impairing cytokinesis of a cell.

By "cytokinesis" it is meant the process in which the cytoplasm of a single mitotic eukaryotic cell is divided to form two daughter cells, i.e. it is the division of the cytoplasm and of membranes. Cytokinesis usually initiates during the late stages of mitosis, and sometimes meiosis, splitting a binucleate cell in two. Failure to achieve cytokinesis may result in multinucleate cells, thereby triggering their death.

In normally functioning cells, Optineurin protein(s) are associated with the Golgi apparatus at all stages of a cell's life to the exclusion of the mitosis stage, during which, upon phosphorylation by PIk1 at amino-acid residue 177, Optineurin protein(s) dissociate(s) from the Golgi apparatus to migrate toward the nucleus of the cell and translocate into said nucleus. The subcellular localization of Optineurin protein(s) is essential for the Optineurin-mediated PIk1 auto-regulation loop pathway.

The term "associated with the Golgi apparatus" encompasses either the fact that a polypeptide of the invention binds the Golgi apparatus or interacts with one or more components at least partly linked to the surface of the Golgi apparatus including Rab8 protein or enters into close vicinity with components at the surface of the Golgi apparatus.

According to a particular embodiment of the invention, use is made of a polypeptide that is capable of disrupting the interaction between endogenous Optineurin protein(s) and Rab8 protein(s) in a mitotic cell.

According to a specific embodiment, when said disruption occurs, the Rab8 protein(s) of the cell remain(s) associated with the Golgi apparatus of said cell and the polypeptide of the invention, which is capable of binding to Rab8 protein(s), substitutes to endogenous Optineurin in its binding to Rab8 protein(s).

Accordingly, dissociated endogenous Optineurin proteins are expected to migrate toward and translocate into the nucleus of said mitotic cell, therefore supplying endogenous Optineurin proteins to the Optineurin-mediated PIk1 negative auto-regulation loop pathway. Such a scheme gives rise to PIk1 inactivation and, through enhancing negative regulation of PIk1 activity attributed to Optineurin, such a scheme might also lead to a failure to complete mitosis, eventually resulting in cell death.

As a consequence, and according to a particular embodiment, using a polypeptide of the invention competing with the binding between the endogenous Optineurin protein(s) and the Rab8 protein(s) on the Golgi apparatus with the view to interfer with the Optineurin-mediated PIk1 auto-regulation loop pathway, enables modulating the subcellular distribution, between the cytoplasm and the nucleus, of the endogenous Optineurin protein in a cell.

According to a particular embodiment, a polypeptide of the invention has a higher affinity for Rab8 protein(s) or fragment(s) thereof, especially when the latter are associated with the Golgi apparatus in a mitotic cell, than a mutated or wild-type endogenous Optineurin protein or fragment thereof.

By "higher affinity" it is meant that the physical value measuring the strength of the association between a polypeptide of the invention and Rab8 protein(s) or fragment(s) thereof demonstrates that this association is stronger than the association between a wild-type endogenous Optineurin protein and Rab8 protein(s).

Moreover, the inventors have shown that migration of wild-type Optineurin protein(s) toward the nucleus of the cell requires prior phosphorylation at its/their position(s) 177, by activated PIk1 protein(s).

Therefore, according to a particular embodiment of the invention, use is made of a polypeptide that can be phosphorylated, especially at a position similar to position S177 by reference to the wild-type human Optineurin sequence, by activated PIk1 protein(s) in a cell. Such a polypeptide competes with endogenous Optineurin for binding to MYPT1 in the MP complex and for phosphorylation, putatively decreasing the pool of endogenous phosphorylated Optineurin available for such molecular interactions.

Accordingly, in a particular embodiment, use is made of a polypeptide that is an Optineurin fragment acting as a PIk1 substrate, meaning that said polypeptide can be phosphorylated by PIk1, especially by activated PIk1.

Inventors also found that Optn functionally connects MYPT1 and PP1β to PIk1 during mitosis by promoting phosphorylation of MYPT1. MYPT1 and PP1β are therefore functional partners in the interaction between Optineurin and Plk1 (MYPT1 and PP1β are generally found associated together in a Myosin Phosphatase (MP) complex). It can be envisioned to either preserve or abolish the interaction(s) of MYPT1 and PP1β with Optineurin and PIk1.

However, within the particular embodiment wherein use is made of a polypeptide that is an Optineurin fragment acting as a PIk1 substrate, such a polypeptide should be rendered capable to interact with MYPT1 protein in the cell, especially MYPT1 protein engaged in a Myosin-Phosphatase (MP) complex, in order to also compete with endogenous Optineurin with respect to the interaction with the MYPT1 partner.

Therefore, according to a particular embodiment, use is made of a polypeptide, which binds MYPT1 protein, when said MYPT1 protein is engaged in a Myosin-Phosphatase (MP) complex, and which is a substrate for PIk1 that can be phosphorylated by activated PIk1 in said cell.

Alternatively, use can be made of a polypeptide that is not capable to be phosphorylated by activated PIk1 in a cell, i.e. a nonphosphorylatable polypeptide, that is not an Optineurin fragment acting as a PIk1 substrate. Use of such a polypeptide would prevent said polypeptide from interfering with further steps of the Optineurin-mediated PIk1 auto-regulation loop pathway. It is expected that such a polypeptide would not migrate into the nucleus, or, if said migration still occur, such a polypeptide would not possess all the necessary properties for the entire Optineurin-mediated PIk1 auto-regulation loop pathway to be completed.

Consistently and preferably within the particular embodiment wherein use is made of a nonphosphorylatable polypeptide, that is not an Optineurin fragment acting as a PIk1 substrate, such a polypeptide should not be rendered capable to interact with MYPT1 protein(s) in the cell, especially MYPT1 protein engaged in a Myosin-Phosphatase (MP) complex, since otherwise the added polypeptide would also compete with endogenous Optineurin with respect to the interaction with the MYPT1 partner.

Therefore, according to a particular embodiment, use is made of a polypeptide, which does not possess the capacity to bind MYPT1 protein in a cell, when said MYPT1 protein is engaged in a Myosin-Phosphatase (MP) complex, and cannot be phosphorylated by activated PIk1 in said cell.

It results from the above disclosure that both uses of a phosphorylatable or an nonphosphorylatable polypeptide allow interfering with the Optineurin translocation or migration process between the cytoplasm and the nucleus of a cell.

As a consequence, a polypeptide of the invention can also be defined as having the capacity of interfering with the PIk1 T210-dephosphorylation process or the PIk1 inactivation cascade associated with the Optineurin translocation from the cytoplasm into the nucleus of a cell.

According to a particular embodiment, the invention enables the induction of mitosis-linked defects, and in particular impairs cytokinesis, and especially prevents the completion of the cytokinesis.

Polypeptide(s) as described herein can therefore be used as a medicament to prevent mitosis completion.

In a particular embodiment, the invention enables the induction and/or production of multinucleate cells.

In another particular embodiment, the invention leads to mitosis arrest in a cell, thereby inducing said cell death.

The invention provides methods which can be performed *in vivo* or *in vitro.*

According to a particular embodiment, the invention is useful for treating mitosis deregulation(s) or alteration(s) in a cell.

The invention also relates to a method for either increasing or decreasing negative regulation of PIk1 activity *in vitro* in a cell, thereby interfering with the cascade of events involved in said cell mitosis, by contacting said cell with a polypeptide that modulates the subcellular distribution between the cytoplasm and the nucleus of the endogenous Optineurin protein in said cell.

According to another particular embodiment, the invention is useful for preventing or treating a disease involving mitosis deregulation(s) or alteration(s), such as cancers, in an animal or human body.

Accordingly, the term "treatment" as used herein is not restricted to curing a disease and removing its causes but particularly covered means to cure, alleviate, remove or lessen the symptoms associated with the disease of interest, or prevent or reduce the possibility of contracting any disorder or malfunction of the host body. Therefore, the expression "treatment" encompasses the curative effect achieved with compounds of the invention and also the beneficial effect for an animal or patient undergoing the treatment, said effect being either obtained at cellular level or clinical level, including as a result, an improvement of the condition of the animal or patient and/or a remission state or a recovery of a health state.

In a particular embodiment, a polypeptide of the invention can be associated, either physically or within a composition or through a separate administration, with additional active compounds useful for the prophylaxis or the treatment of tumors, either general compounds or compounds proved to be active in a tissue-specific cancer. In a particular embodiment, a polypeptide of the invention can be used as a complement to radiotherapy treatment(s), such as radiotherapy treatment(s) involving DNA lesions, and/or as a complement to chemotherapy(ies). Accordingly a polypeptide of the invention can be used in an adjuvant therapy of pathologies such as cancers or adjuvant therapy when administered treatment gives rise to DNA lesions.

According to a particular embodiment or additionally, the invention can enable to further increase cell(s) death in an animal or human body subject to a disease involving mitosis deregulation.

The invention also relates to antibodies, either as found in a polyclonal serum obtainable by the immunization of an animal or a human with a polypeptide of the invention or monoclonal antibodies directed against a polypeptide of the invention.

According to a preferred embodiment, an antibody as found in a polyclonal serum or a monoclonal antibody of the invention recognizes a polypeptide that discloses a PIk1 phosphorylation consensus sequence D/E-X-S-φ-X-D/E (X, any amino acid; φ, a hydrophobic amino acid), and which is phosphorylated within this consensus sequence, especially at the position of the Serine amino-acid residue.

According to a specific embodiment, an antibody in a polyclonal serum or a monoclonal antibody of the invention recognizes a polypeptide that is phosphorylated at position 177 by reference to the Optineurin protein sequence SEQ ID N°1.

In a particular embodiment, an antibody of the invention recognizes a phosphorylated epitope comprising any one of the sequences SEQ ID N°4 to SEQ ID N°8.

The invention thus also relates to the use of antibody(ies) of the invention, as a probe or marker targeting, for visualization, phosphorylated epitope(s) susceptible to interact with PIk1 within the Optineurin-mediated PIk1 auto-regulation loop pathway.

Use of such an antibody can also allow for staining cell(s) or tissue(s) in ex vivo, specifically in *in vitro* experiments.

In a particular embodiment, such antibody(ies) are used in a *in vitro* screening method for identifying compound(s) capable of specifically activating the phosphorylation of endogenous Optineurin, or enhancing endogenous Optineurin translocation into the nucleus of a cell, thereby negatively regulating PIk1 activity in a cell, comprising the steps of:
i. contacting a cell with an antibody specifically recognizing either the Optineurin protein or an Optineurin-mimicking polypeptide with a PIk1 phosphorylation consensus sequence D/E-X-S-φ-X-D/E (X being any amino acid residue; φ being a hydrophobic amino acid residue) whose Serine amino-acid residue is phosphorylated, especially an Optineurin-mimicking polypeptide that is phosphorylated at position 177 by reference to the Optineurin protein sequence SEQ ID N°1,
ii. contacting said cell with compound(s) to assay, wherein steps i. and ii. can be inverted,
iii. visualizing the change(s) in properties of the cell contacted in steps i) and ii), and,
iv. optionally, recording or quantifying the change(s) in properties of the cell contacted in steps i) and ii), and,
v. optionally, detecting the activity of PIk1, in particular detecting the phosphorylation on Threonine 210 residue of PIk1.

According to a particular embodiment, a further step aimed at determining whether the assayed compound is capable of negatively regulating PIk1 activity within said cell is added.

By "specifically recognizing either the Optineurin protein or an Optineurin-mimicking polypeptide with a PIk1 phosphorylation consensus sequence D/E-X-S-φ-X-D/E", antibody(ies) recognizing either a Optineurin protein or an Optineurin-mimicking polypeptide that is mutated in order to artificially bear several amino-acid residues that can be phosphorylated in the vicinity of a PIk1 phosphorylation consensus sequence D/E-X-S-φ-X-D/E (X being any amino acid residue; φ being a hydrophobic amino acid residue) is/are excluded from the presently disclosed invention.

Similar *in vitro* screening methods can also be performed for testing any variable susceptible of having a measurable effect on an assayed cell, including by contacting organic or chemical compounds, especially drugs, with cells. Such a screening method might allow targeting compounds improving the nuclear localization of Optineurin in a cell, expectedly increasing PIk1 activity negative regulation.

Antibody(ies) of the invention used in a *in vitro* screening method of the invention can be labelled, especially in a view of using imaging readers or devices, for example high-throughput imaging readers for collecting information from assayed cells.

The invention seeks to take advantage of the use of polypeptide(s) as described herein to impact the Optineurin-mediated PIk1 auto-regulation loop pathway, for therapeutic purposes. The invention encompasses the fact of either blocking said Optineurin-mediated PIk1 auto-regulation loop pathway or impacting the dynamics of said pathway, either definitively or temporarily.

The invention also relates to composition(s) comprising polypeptide(s) of the invention, in particular pharmaceutical composition(s), said composition(s) comprising if necessary pharmaceutically acceptable excipient(s), such as carrier(s) and/or adjuvant(s).

According to a particular embodiment, the polypeptide(s) and composition(s) described herein are used in a method of therapy practised on human or animal body(ies), in particular for treating a disease involving mitosis deregulation(s) or alteration(s), or its symptom(s).

According to a particular embodiment, the polypeptide(s) and composition(s) described herein are used for treating neoplasic disease(s), including carcinoma(s), melanoma(s), myeloma(s), sarcoma(s), leukemia and cancer(s).

It is especially provided that in a particular embodiment of the invention, the molecule(s) of the invention is/are suitable for the treatment of infiltrating or vascularized tumors or superficial tumors or for the treatment of primary or metastatic tumors, in accordance with the acknowledged clinical criteria for the classification of tumors.

Among tumors, which may be candidates for the treatment with the polypeptide(s) of the invention, the following are described as examples:

Melanoma; Lung carcinoma; Head & neck carcinoma; cervical carcinoma, Esophageal and gastric carcinoma; Bladder carcinoma, especially infiltrating Bladder carcinoma; Prostate carcinoma; Breast carcinoma; Colorectal adenocarcinoma; Renal cell carcinoma; Sarcoma; Leukemia; Myeloma, thyroid cancer, gliomas, urethelial carcinoma.

According to a particular embodiment, the polypeptide(s) and composition(s) described herein are used for treating Optineurin-mutation(s) linked diseases, primary open-angle glaucoma, juvenile open-angle glaucoma, amyotrophic lateral sclerosis, Huntington disease.

According to a particular embodiment all polypeptide(s), composition(s), nucleic acid molecule(s) or vector(s) disclosed herein can be used in a therapy method as disclosed herein, or for any purpose disclosed herein.

The invention also relates to a kit comprising a polypeptide or a composition of the invention for use either to increase or to decrease negative regulation of PIk1 activity in a cell, thereby impairing or therapeutically interfering with the cascade of events involved in said cell mitosis, optionally comprising appropriate buffer(s) and/or reagent(s) and/or adjuvant(s) for achieving said increase or decrease of the negative regulation of PIk1 activity in a cell, or necessary for carrying out such a process.

Said kits can further comprise instructions for use in a method for increasing or to decreasing negative regulation of PIk1 activity in a cell, according to a method of the invention or an use of a compound or composition of the invention as disclosed herein.

Said kits can further comprise material, e.g measurement material, data carrier(s), recording support(s), to collect or analyze the data measured by a method or an use according to the invention.

Other examples and features of the invention will be apparent when reading the examples and the figures, which illustrate the experiments conducted by the inventors, in complement to the features and definitions given in the present description.

### FIGURES LEGENDS

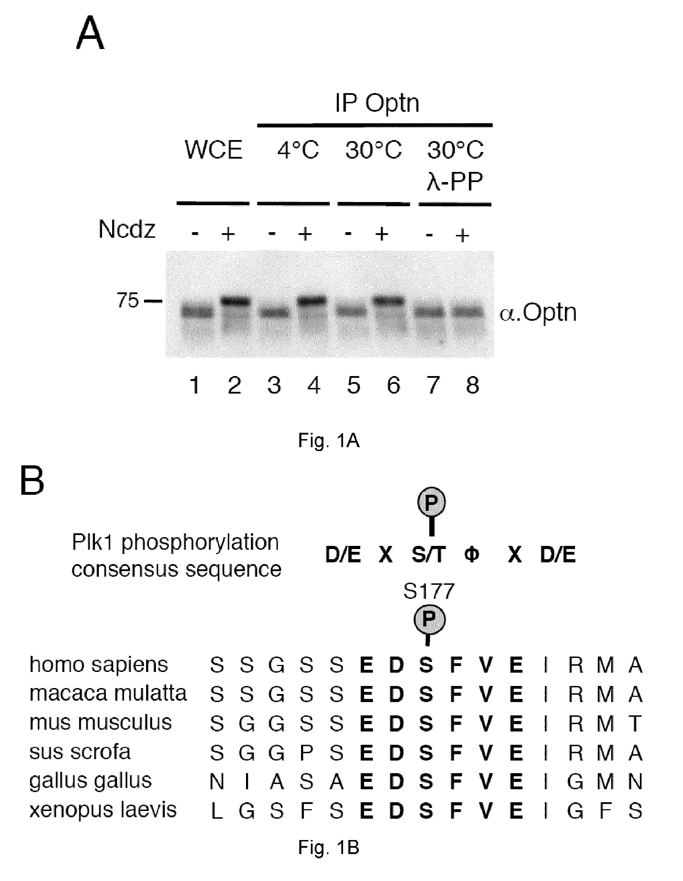

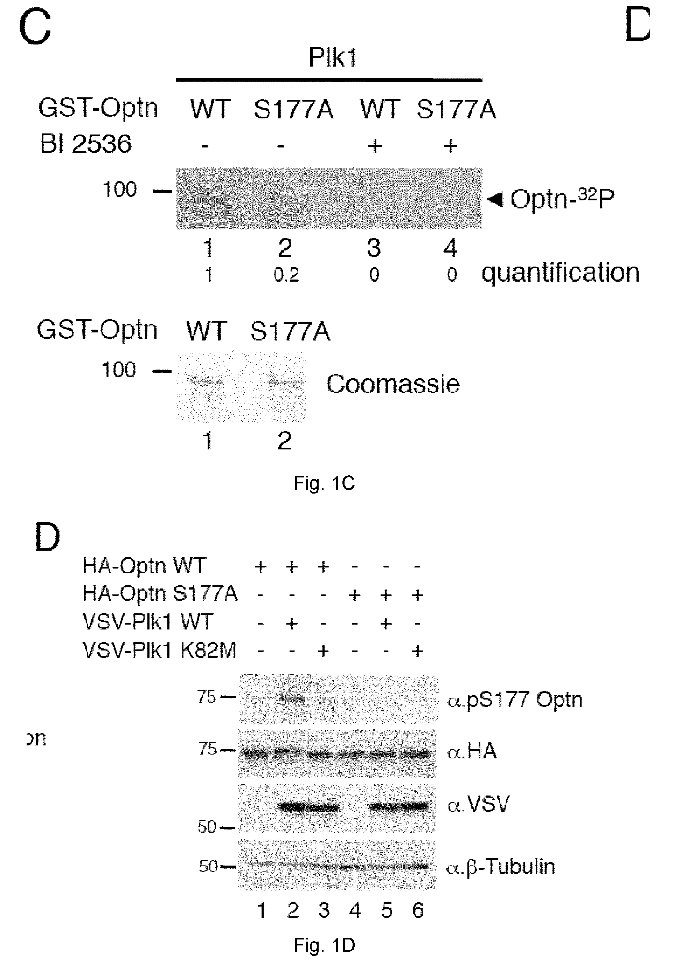

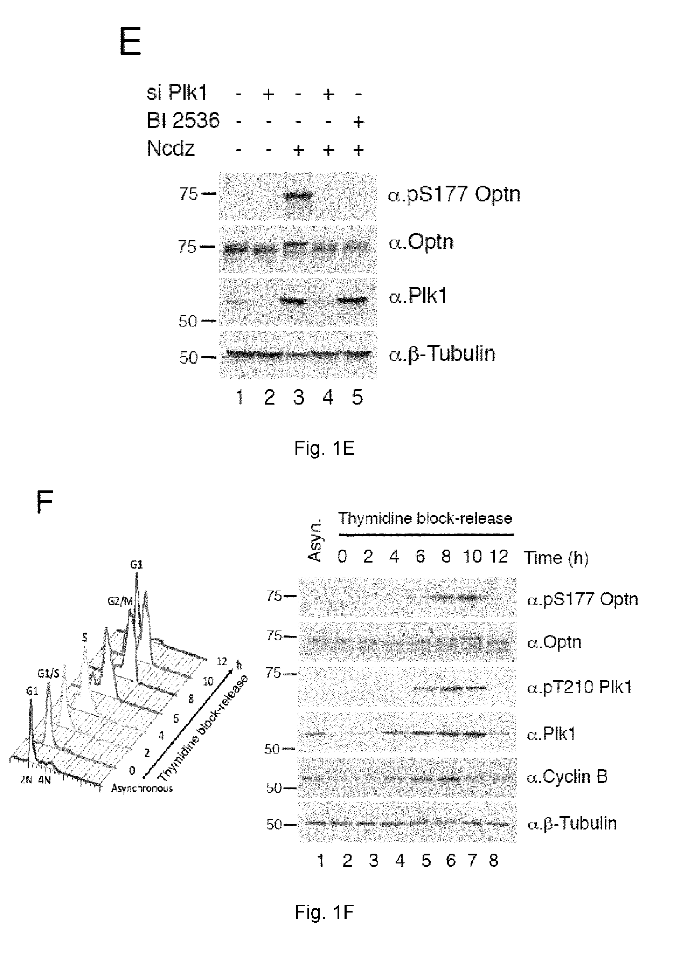

**Figure 1****: Optn is phosphorylated on serine 177 by PIk1 during mitosis**
   (A) Whole cell extracts (WCE) or Optineurin immunoprecipitates (IP Optn) from asynchronous or mitotic cells (treated with Nocodazole: Ncdz) were incubated at the indicated temperature with or without lambda-phosphatase (I-PP). Optn is detected by Western blotting with an anti-Optn antibody.
   (B) Alignment of Optn protein sequences from different species reveals a conserved serine residue within a consensus motif for phosphorylation by PIk1.
   (C) *In vitro* kinase assays were performed in the presence of recombinant constitutively active PIk1 and GST-Optn, either wild type (WT) or mutated on Ser177 (S177A). BI2536 was added as indicated. Kinase reactions were analyzed by autoradiography and phosphorylation levels of GST-Optn were quantified relative to lane 1.
   (D) HeLa cells were transfected with the indicated plasmids (PIk1 K82M is a kinase dead form of PIk1), lysed and protein extracts were analyzed by Western Blot using the indicated antibodies.
   (E) HeLa cells were transiently depleted of PIk1 using siRNA, and treated with Nocodazole and BI 2536 as indicated, lysed and protein extracts were analyzed by Western Blot using the indicated antibodies.
   (F) HeLa cells were arrested at the G1/S boundary with a double thymidine block and then released into fresh medium. Cells were collected every 2 hours and stained with propidium iodide. The cell-cycle profile was determined by flow cytometry (**left panel**) and the indicated proteins were assayed by Western blotting (**right panel**). "Asyn." indicates asynchronous cells; 2N and 4N represent DNA content.

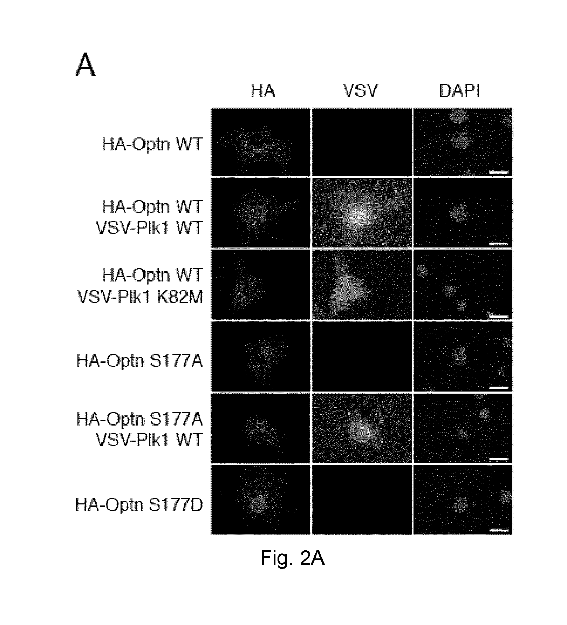

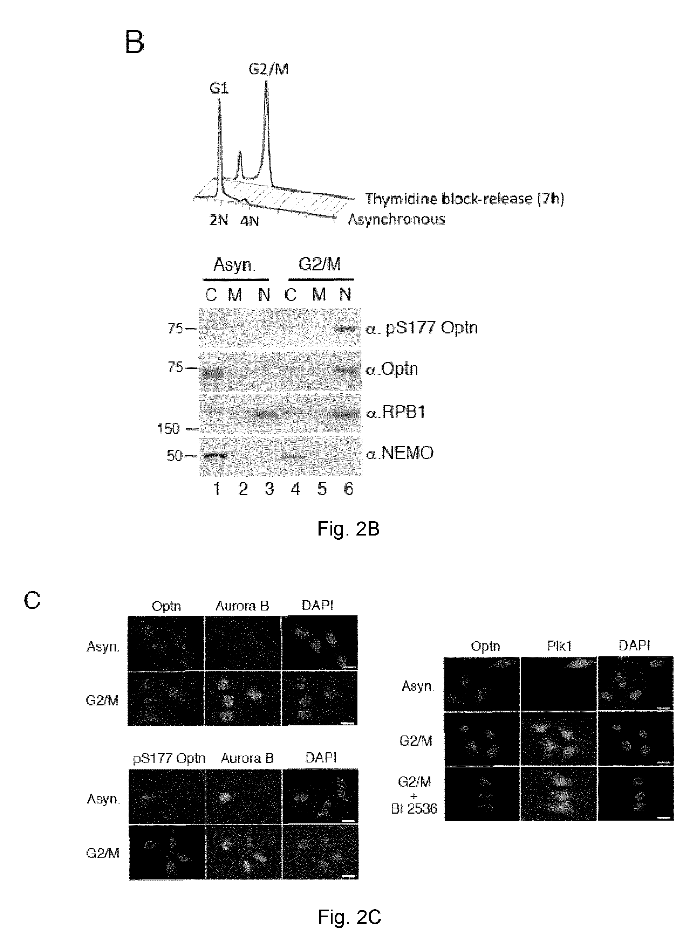

**Figure 2****: Optn translocates into the nucleus during mitosis upon phosphorylation by PIk1**
   (A) Cos-7 cells were transfected with the indicated plasmids, and stained for HA (red), VSV (green) and DNA (blue). Scale bar 10mM.
   (B) HeLa cells were arrested at the G1/S boundary with a double thymidine treatment and then released for 7 hours. The cell cycle profile was determined by flow cytometry (**Top panel**). HeLa cells were fractionated into cytoplasmic (C), membrane (M) and nuclear (N) fractions (**Bottom panel**). Western blot were performed using the indicated antibodies.
   (C) HeLa cells were synchronized in G2/M by a double thymidine block followed by a release for 7 hours, and were treated with BI 2536 as indicated and stained for Optn or pS177 Optn (red - left columns), Aurora B or PIk1 (green - middle columns) and DNA (blue - right columns). In asynchronous cells, the nuclear localization of Optn and its phosphorylated product is only detected in mitotic cells which are also positive for the presence of Aurora B (left panel) or PIk1 (right panel). Scale bar 10 µM.

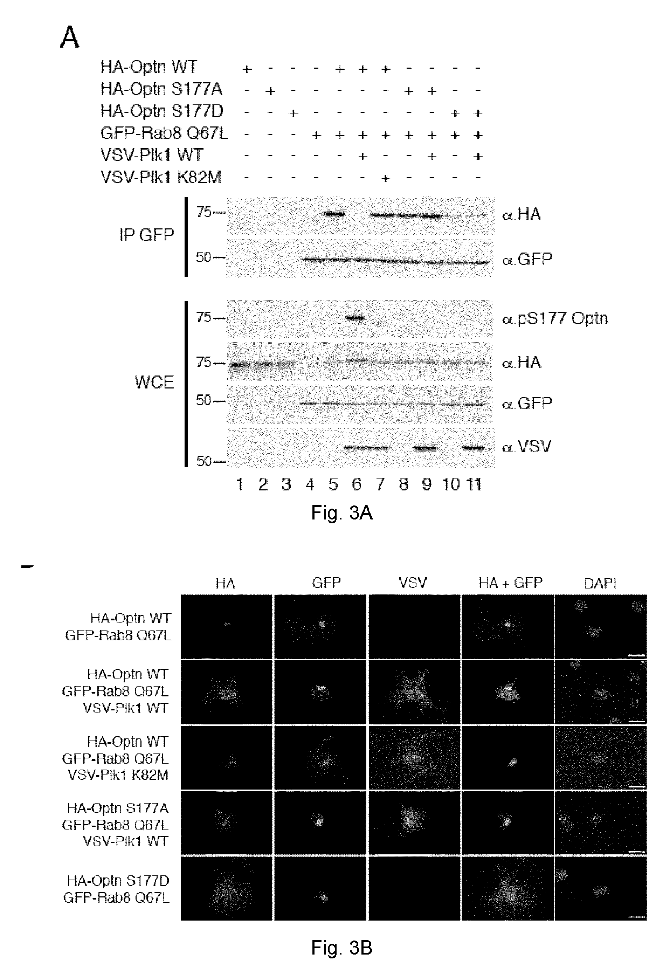

**Figure 3****: Phosphorylation of Optn by PIk1 prevents its association with Rab8 and induces its nuclear translocation**
   (A) HeLa cells were transfected with the indicated plasmids and cell lysates were subjected to immunoprecipitation with anti-GFP antibodies and immunoblotted with the indicated antibodies.
   (B) HA-Optn WT and mutants (red) were transiently coexpressed in Cos-7 cells with GFP-Rab8 Q67L (green), either alone or with PIk1 WT or K82M (magenta), and analyzed by immunofluorescence. DNA is stained with DAPI (blue) and colocalization between HA and GFP is shown in yellow. Scale bar 10 µM.

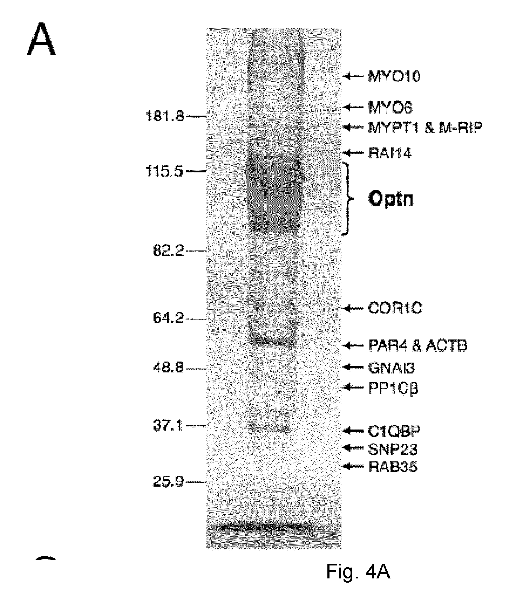

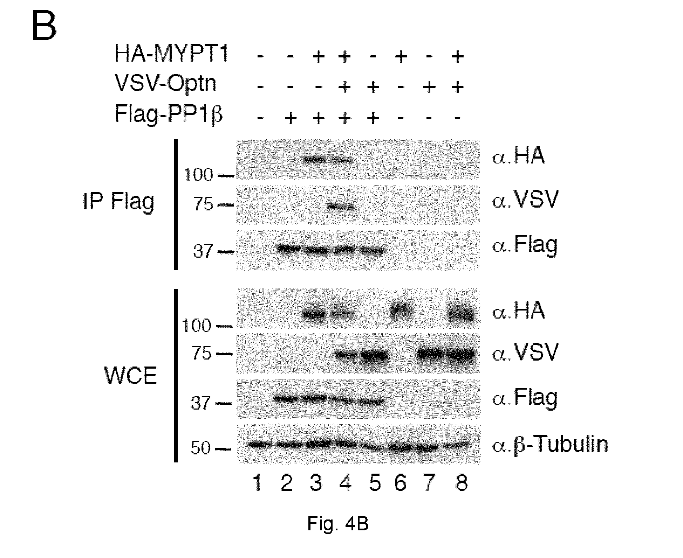

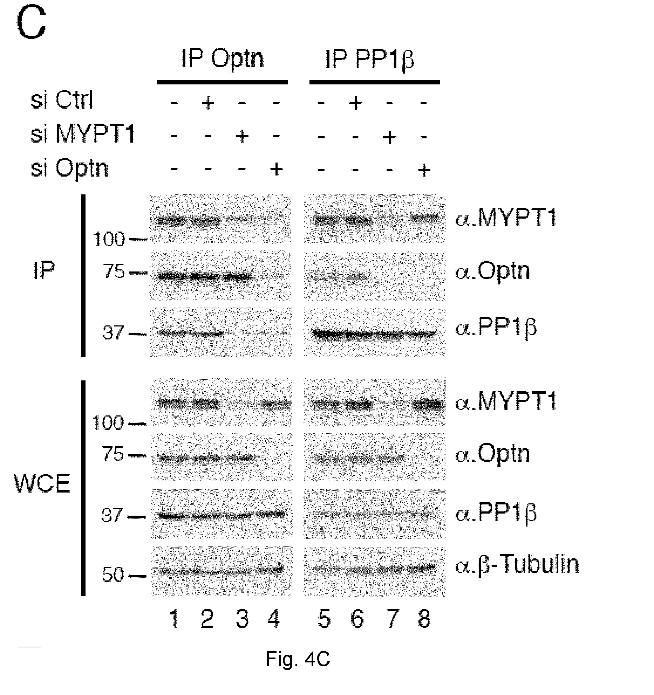

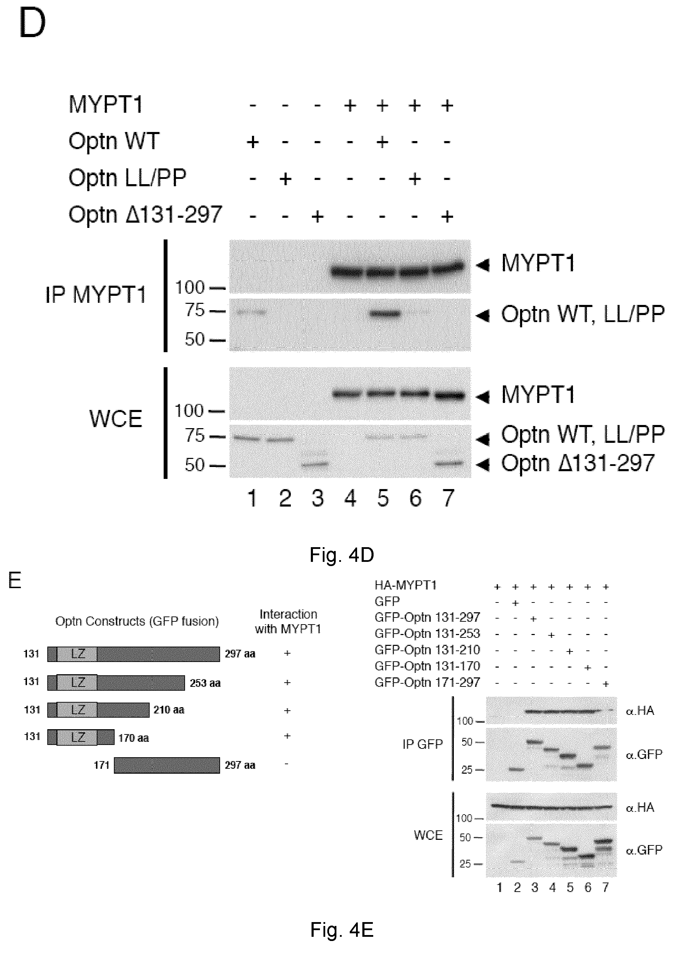

**Figure 4****: Optineurin interacts with MYPT1 and PP1β**
   (A) Protein complexes associated with GS-TAP-tagged Optn were isolated by tandem affinity purification, separated by SDS-PAGE and visualized by silver staining. Protein complex composition was analyzed by LC-MSMS. The position of several complex components is depicted next to the region where it was identified.
   (B) HEK-293T cells were transfected with the indicated plasmids. Total lysates were immunoprecipitated with an anti-Flag antibody and immunoblotted with the indicated antibodies
   (C) HeLa cells were transfected with either an irrelevant siRNA (Ctrl) or Optn- or MYPT1-directed siRNA. Total lysates were immunoprecipitated with anti-Optn or anti-PP1β antibodies. Western blot analysis were performed using the indicated antibodies.
   (D) HeLa cells were transfected with HA-MYPT1 and either HA-Optn WT, HA-Optn D131-297 (deletion aa 131-297) or HA-Optn LL/PP (substitution of leucine 150 and 157 by proline residues). Lysates were immunoprecipitated with an anti-MYPT1 antibody and Western blot analysis were performed using anti-HA antibody.
   (E) The putative Leucine Zipper of Optn is sufficient for the interaction with MYPT1. Left panel: Schematic representation of Optn constructs and their ability to interact with MYPT1. Right panel: Optn fragments fused to GFP and HA-MYPT1 were transiently co-expressed in HeLa cells (**Right panel**). Lysates were immunoprecipitated with an anti-GFP antibody and Western blot analysis were performed with anti-HA and anti-GFP antibodies.

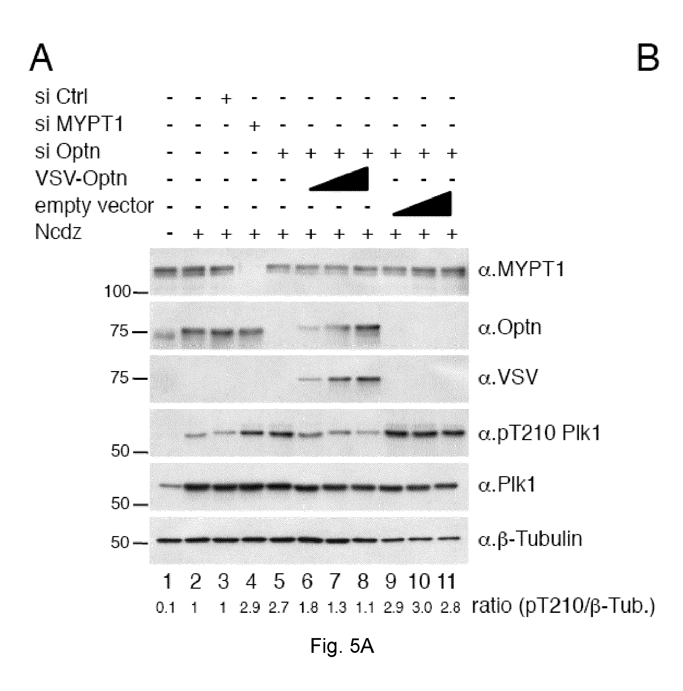

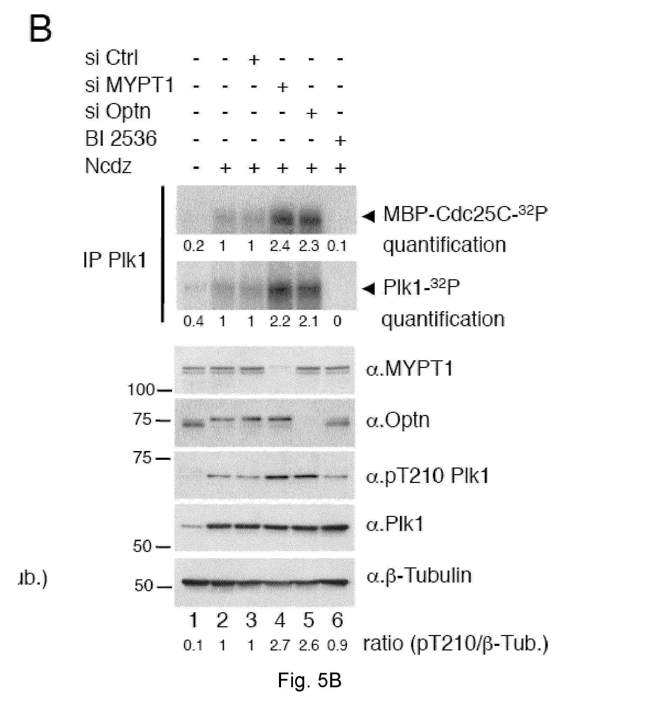

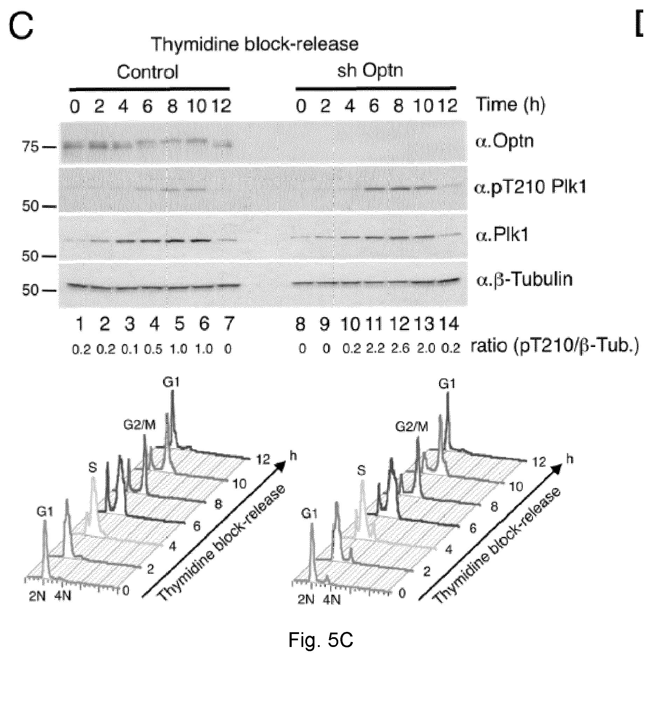

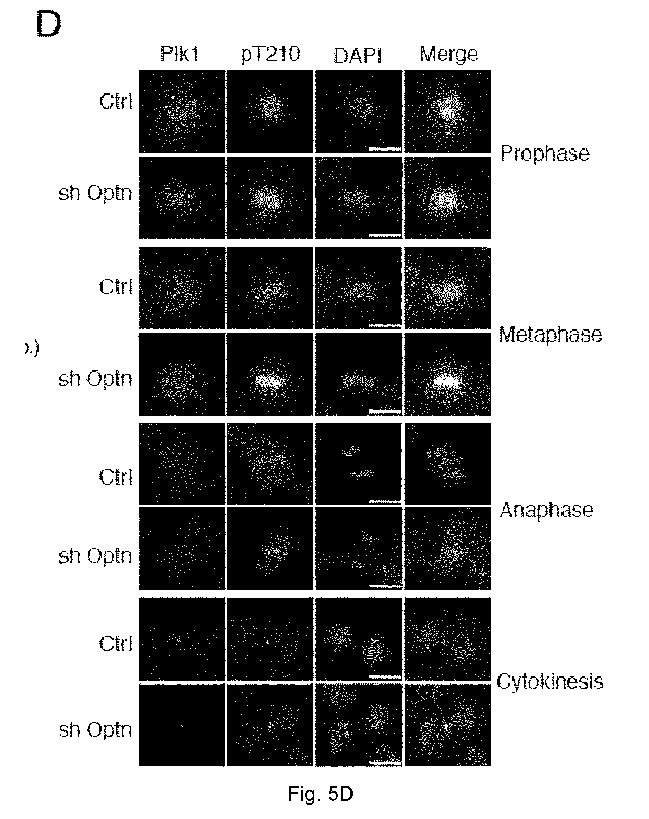

**Figure 5****: Optn regulates PIk1 phosphorylation and activity during mitosis**
   (A) HeLa cells transfected with the indicated plasmids and siRNAs were treated with Nocodazole as indicated. The fold induction of PIk1 phosphorylation is calculated as a ratio between pT210 and β-Tubulin signals measured with the Quantity one software.
   (B) HeLa cells were transiently transfected with the indicated siRNA and treated with Nocodazole and BI 2536 as indicated. PIk1 was immunoprecipitated and incubated in kinase reaction buffer in the presence of ³²P-g-ATP and recombinant MBP-Cdc25C. Phosphorylation of PIk1 and MBP-Cdc25C were analyzed and quantified by autoradiography relative to signal obtained in Ncdz-treated cells (lane 3). Western blots of whole cell lysates were performed using the indicated antibodies and PIk1 phosphorylation was quantified as in panel A.
   (C) HeLa cells clones stably depleted for Optn (sh Optn) or stably transfected with the empty vector (Control) were synchronized at the G1/S boundary by double thymidine block, released into fresh medium, and the experiment was performed as in Figure 1F.
   (D) Immunofluorescence experiments show the locations of PIk1 (red) during mitosis and the intensity of PIk1 phosphorylation (pT210, green). DNA is stained with DAPI (blue), colocalization of PIk1 with pT210 is shown in yellow. Scale bar 10 µM.

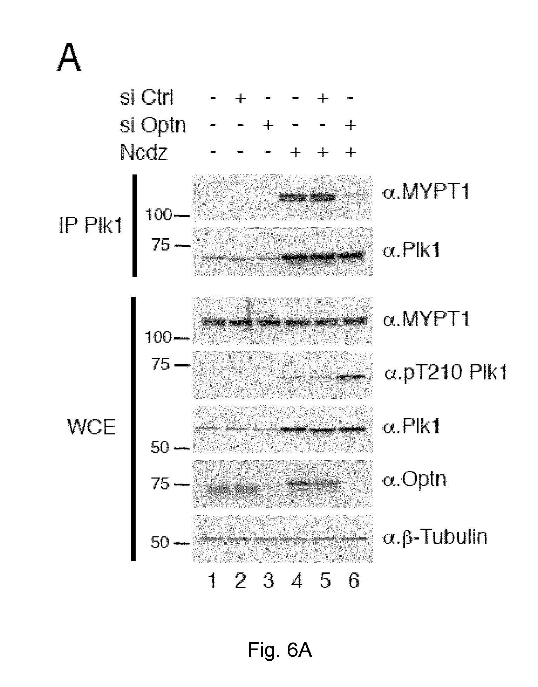

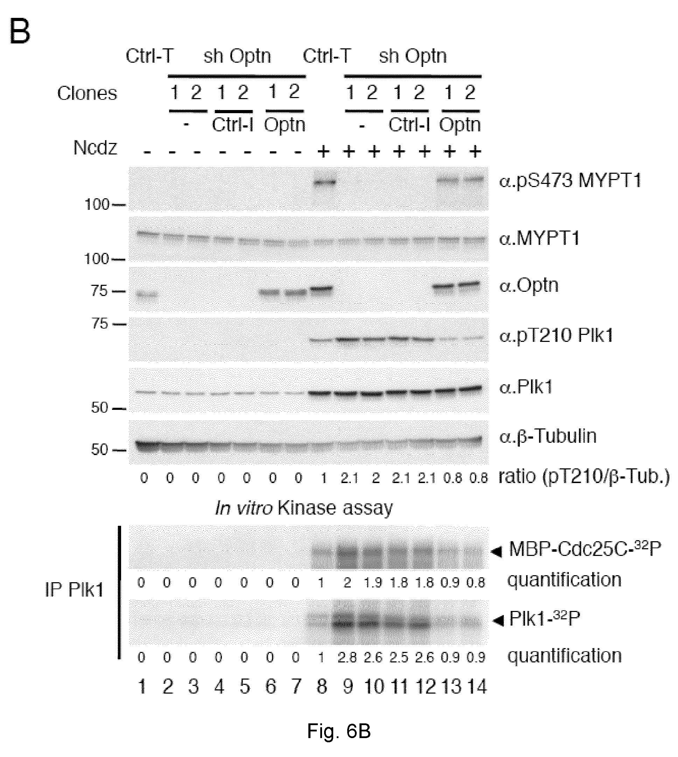

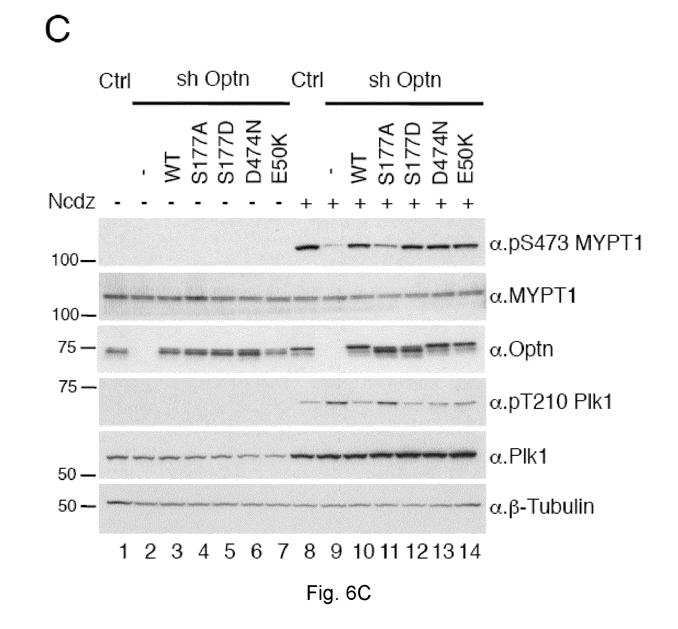

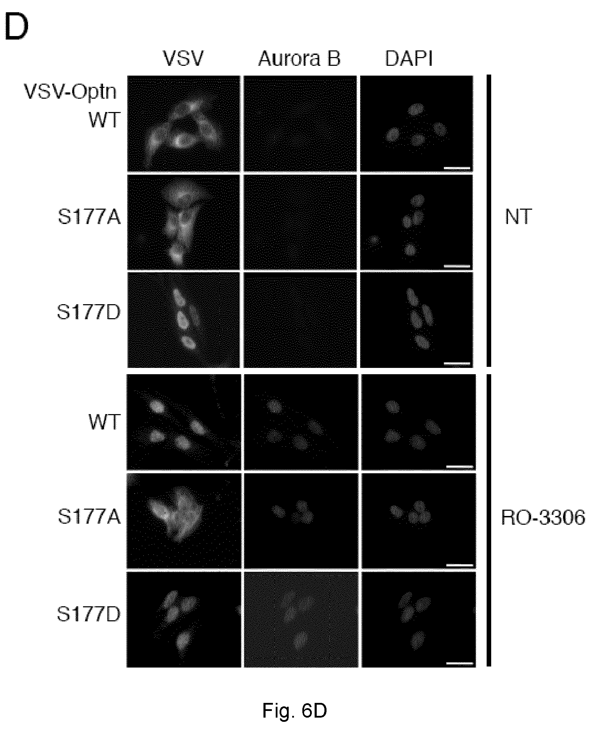

**Figure 6****: The translocation of Optn into the nucleus promotes the phosphorylation of MYPT1 in order to antagonize PIk1 activity.**
   (A) HeLa cells were transiently transfected with the indicated siRNAs and treated with Nocodazole as indicated. Lysates were immunoprecipitated with an anti-PIk1 antibody. Western blots were performed using the indicated antibodies.
   (B) HeLa cells were stably transfected with the empty vector (Ctrl-T) or with a plasmid expressing Optn-directed shRNA. Optn depleted clones (clone 1 and 2) were infected with empty retroviruses (Ctrl-I) or retroviruses expressing shRNA-insensitive Optn and treated with Nocodazole as indicated. Levels of pT210 and B-tubulin were analyzed by Western blot and quantified as in Figure 5 (top panel). *In vitro* kinase assays were performed and quantified as in figure 5B (bottom panel).
   (C) Optn-depleted HeLa cells reconstituted with shRNA-insensitive Optn mutants were analyzed by Western blot using specific antibodies against phosphorylated forms of MYPT1 or PIk1.
   (D) Subcellular localization of Ser177 phospho-mutants of Optn (green) was determined in asynchronous (NT) or RO-3306 treated HeLa cells. Aurora B staining (red) in the nucleus indicates cells in late G2 phase. Scale bar 10 µM.

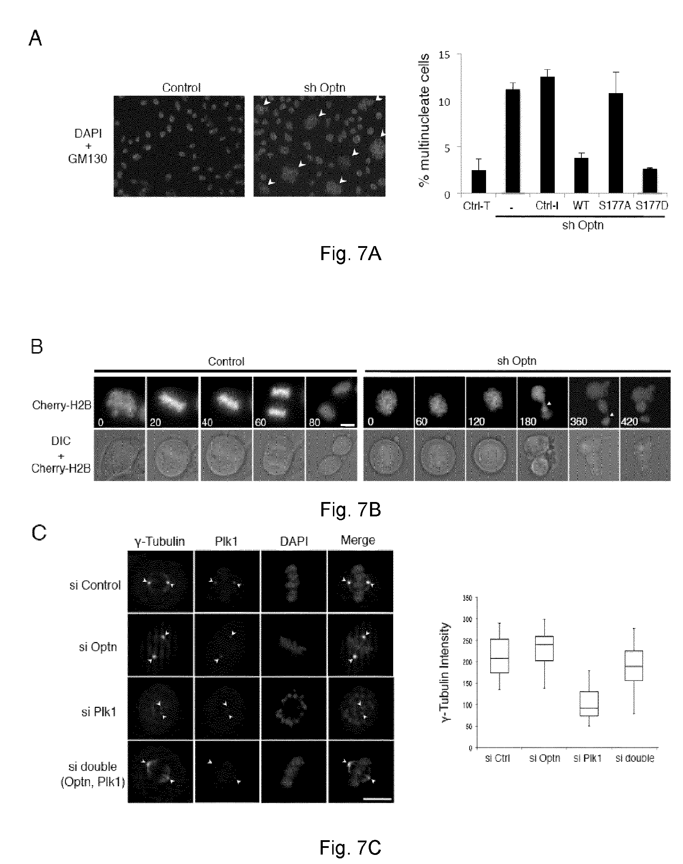

**Figure 7****: Optn depletion induces mitotic defects and antagonizes PIk1 function**
   (A) HeLa clones were fixed, DNA was stained with DAPI (blue) and the Golgi apparatus stained with the Golgi marker GM130 (red) and visualized by fluorescence microscopy. Multinucleated cells are indicated by arrowheads **(left panel)**. Percentage of multinucleate cells were counted for each condition (n=500, repeated 2 times). Ctrl-T: HeLa cells transfected with an empty vector; Ctrl-I: Optn-depleted cells transduced with an empty retroviral vector; WT, S177A and S177D : Optn-depleted cells reconstituted with shRNA-insensitive WT or mutant Optn **(right panel)**.
   (B) Live-Cell Imaging of Control and Optn-depleted HeLa cells. Control cells and Optn depleted cells were transiently transfected with a plasmid encoding Cherry-Histone 2B and imaged using a Axio Observer Z1 microscope system. Images were taken at 2 min intervals. The Cherry fluorescence and the corresponding differential interference contrast images are shown. DNA bridges are indicated by arrowheads. Scale bar 10 µM.
   (C) Localization of γ-Tubulin (green), PIk1 (red) and DNA (blue) in control, Optn-depleted, PIk1-depleted and double (Optn, PIk1)-depleted HeLa cells (**left panel**). Arrowheads indicate centrosomes. Scale bar 5 µm. Box plot of γ-Tubulin intensities of centrosomes (**right panel**). The upper and lower edge of each box represents upper and lower quartiles, respectively. The median is indicated by a horizontal line.

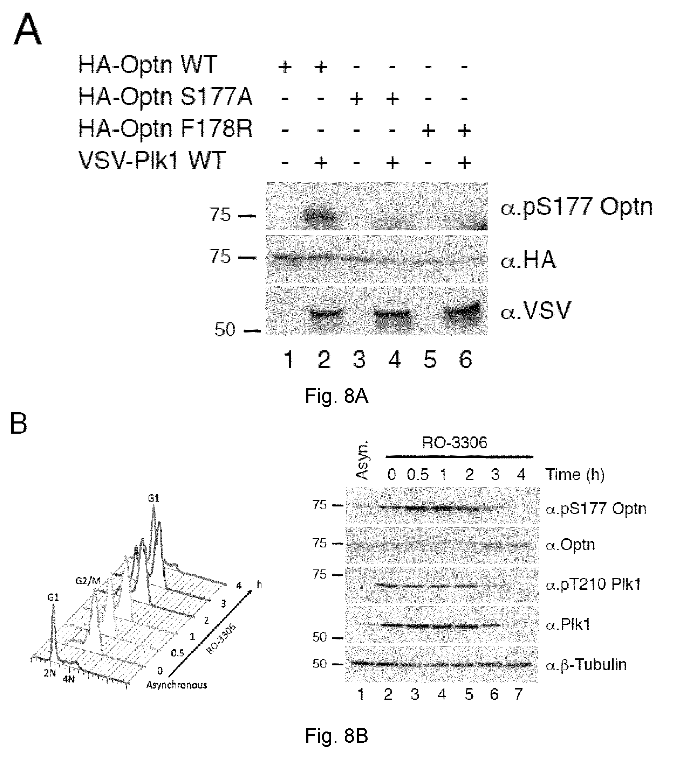

**Figure 8****: Optn is phosphorylated on Serine 177 during mitosis**
   (A) HeLa cells were transfected with the indicated plasmids, lysed and protein extracts were analyzed by Western blot using the indicated antibodies.
   (B) Endogenous Optn is phosphorylated on Ser177 during G2/M phase and coincided with PIk1 expression and activation. HeLa cells were synchronously released from a Cdk1-inhibitor treatment (RO-3306) for the indicated periods of time. At each time point, half of the cells were harvested, lysed and analyzed for Optn and PIk1 phosphorylation and expression by Western blot using the indicated antibodies (**right panel**), whereas the second half was fixed in ethanol and used for cytometric analysis of the cell cycle progression. 2N and 4N represent DNA content (**left panel**).

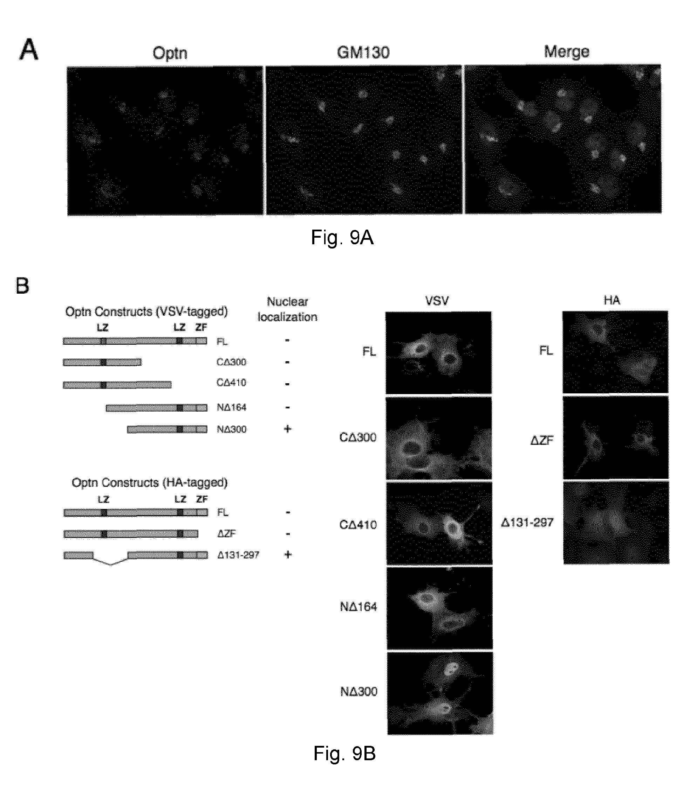

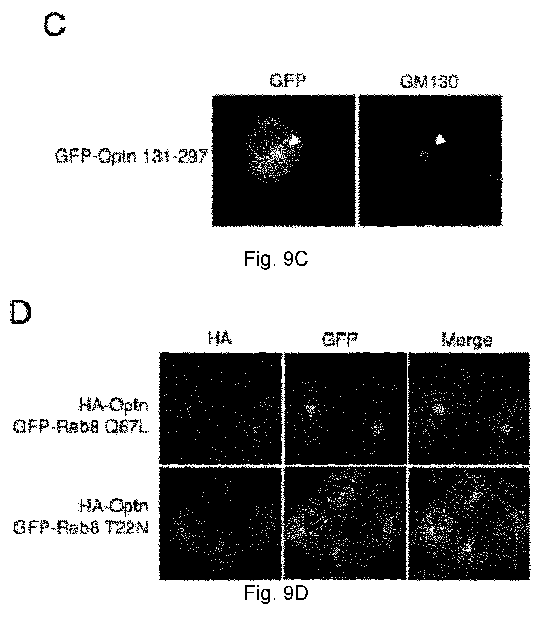

**Figure 9****: Subcellular distribution of Optn WT and mutants**
   (A) Localization of Optn at the Golgi apparatus. Cos-7 cells were fixed and processed for immunostained using anti-Optn or anti-GM130 antibodies. Stained cells were examined by immunofluorescence microscopy. Yellow colour in the merged images indicated co-localization.
   (B) Schematic representation of Optn constructs and their ability to localize into the nucleus (**left panel**). Representative microscopy images of the localization of VSV- and HA-tagged Optn mutants in HeLa cells respectively shown in green and red (**middle and right panels**).
   (C) The [131-297] region is sufficient to target GFP at the Golgi apparatus. HeLa cells were transfected with Optn 131-297 fused to GFP and stained for the Golgi marker GM130 (red).
   (D) Rab8 Q67L expression induced the localization of Optn in tight perinuclear spots. HeLa cells were cotransfected with HA-tagged Optn and either GFP-Rab8 Q67L (dominant active form of Rab8) or GFP-Rab8 T22N (dominant inactive form of Rab8) and stained for HA (red) and DNA (blue). Colocalization appears in yellow.

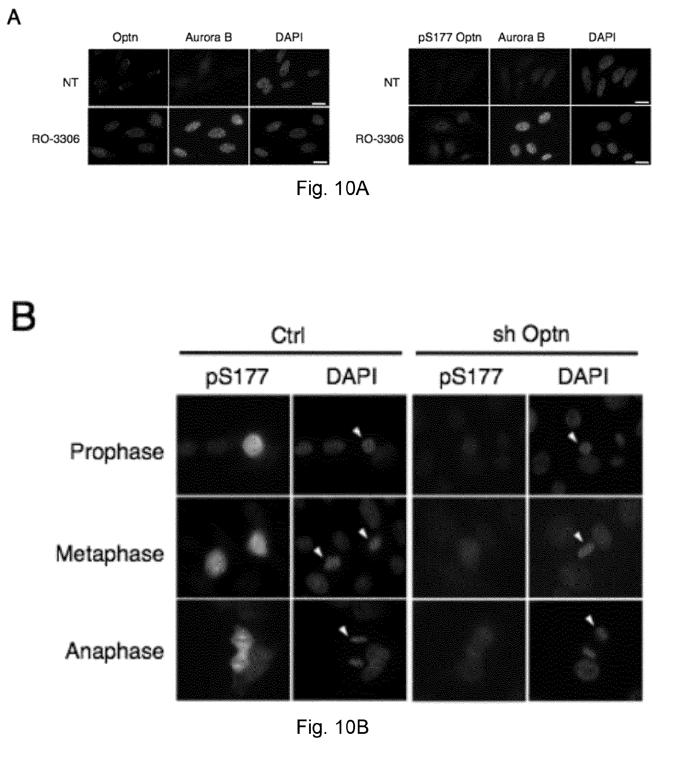

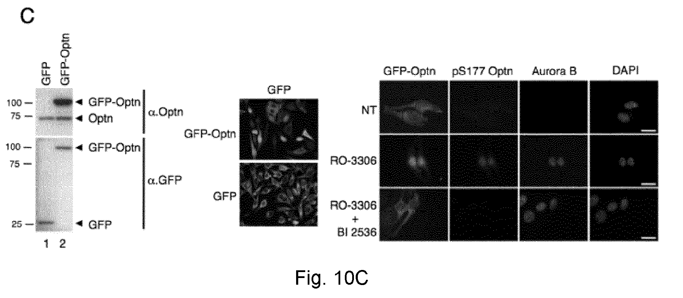

**Figure 10****: Optn translocates into the nucleus during mitosis**
   (A) Optn (**left panel**) and its phosphorylated form (right panel) translocate into the nucleus during the G2 phase. HeLa cells were treated with RO-3306 as indicated and stained for Optn or pS177 Optn (Red), Aurora B (green) and DNA (blue). Scale Bar 10 µm.
   (B) Specificity of the pS177 Optn antibody. Asynchronous HeLa cells stably transfected either with the empty vector or with Optn shRNA were stained for pS177 Optn (green) and DAPI (blue). Representative cells from different stages of mitosis are shown.
   (C) GFP-Optn was stably expressed in HeLa cells and sub-cloned under limiting dilutions. The level of expression of GFP and GFP-Optn were monitored using anti-GFP antibody and were compared to the expression of endogenous Optn by immunoblotting with anti-Optn antibody (**left panel**). GFP and GFP-Optn subcellular localizations in asynchronous cells were visualized by immunofluorescence (**middle panel**). The redistribution of GFP-Optn in G2/M synchronized cells was monitored after treatment of the cells with RO-3306 and BI 2536 as indicated (**right panel**). The anti-pS177 (red) was used to detect phosphorylated Optn. Aurora B staining (magenta) was used to detect cells in late G2 phase. "NT" indicates non-treated cells. Scale bar 10 µM.
**Figure 11** **: Optn dissociates from Rab8 upon phosphorylation by PIk1 on Serine 177**. HEK-293T cells were transfected with the indicated plasmids and cell lysates were subjected to immunoprecipitation with anti-GFP antibodies and immunoblotted with the indicated antibodies.
**Figure 12****: The interaction between endogenous Optn and PP1β is dependent on its association with MYPT1.**
   HEK-293T cells were transfected with either an irrelevant siRNA (control) or Optn- or MYPT1-directed siRNA. Total lysates were immunoprecipitated with an anti-Optn antibody or with an anti-PP1β antibody (or a preimmune antibody, Po). Western blot analysis of immunoprecipitates and whole cell extracts (WCE) were performed using anti-MYPT1, anti-Optn and anti-PP1β antibodies. Anti-β-Tubulin was used as a loading control.

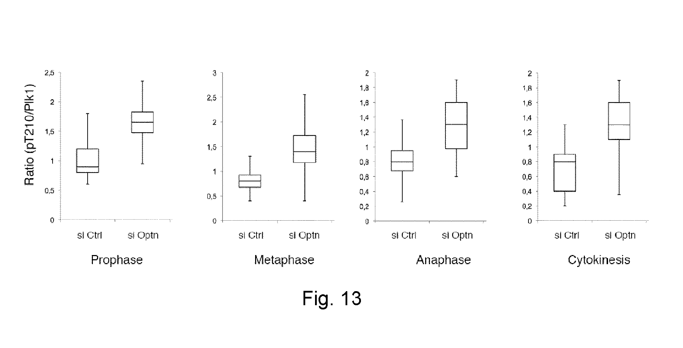

**Figure 13****: Box plot revealing an increase in Thr210 phosphorylation by Optn depletion.**
   Ratios (pT210/PIk1) of staining intensities in control (n=80) and Optn depleted cells (n=80) were analyzed from prophase to cytokinesis. The upper and lower edge of each box represents upper and lower quartiles. The median is indicated by a horizontal line.

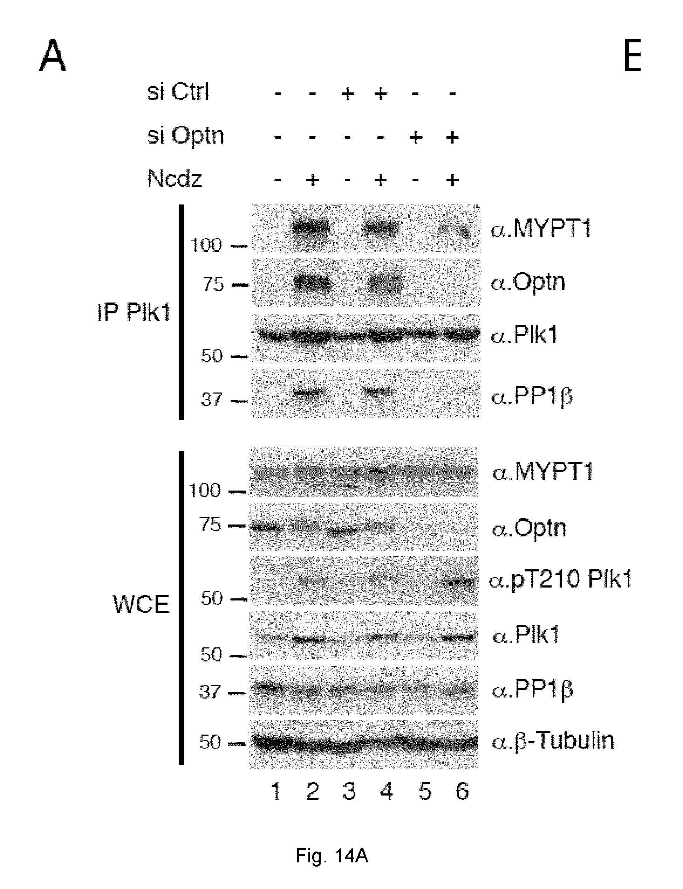

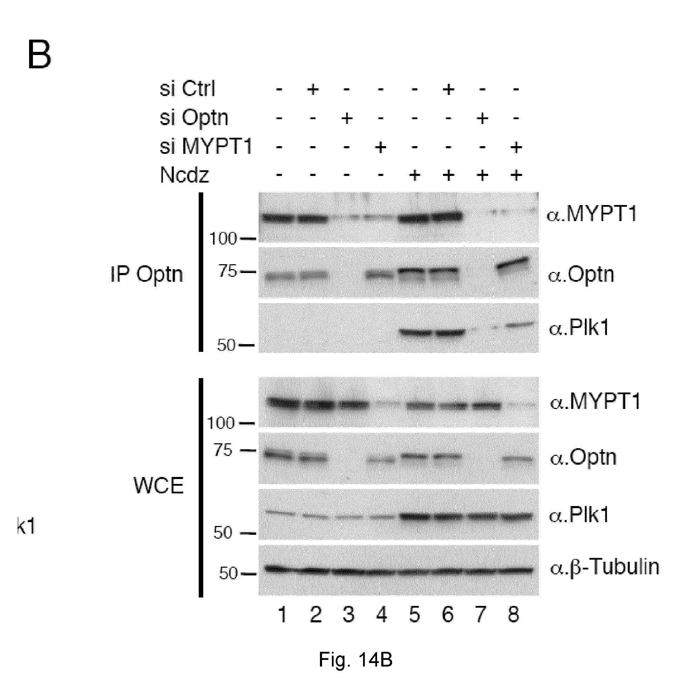

**Figure 14****: Characterization of the interactions involved in the formation of the PIkl-MYPT1-Optn complexes**
   (A) HEK-293T cells were transiently transfected with the indicated siRNAs and treated with Nocodazole as indicated. Lysates were immunoprecipitated with an anti-PIk1 antibody. Western blots were performed using the indicated antibodies.
   (B) HeLa cells were transiently transfected with the indicated siRNAs and treated with Nocodazole as indicated. Lysates were immunoprecipitated with an anti-Optn antibody. Western blots were performed using the indicated antibodies.

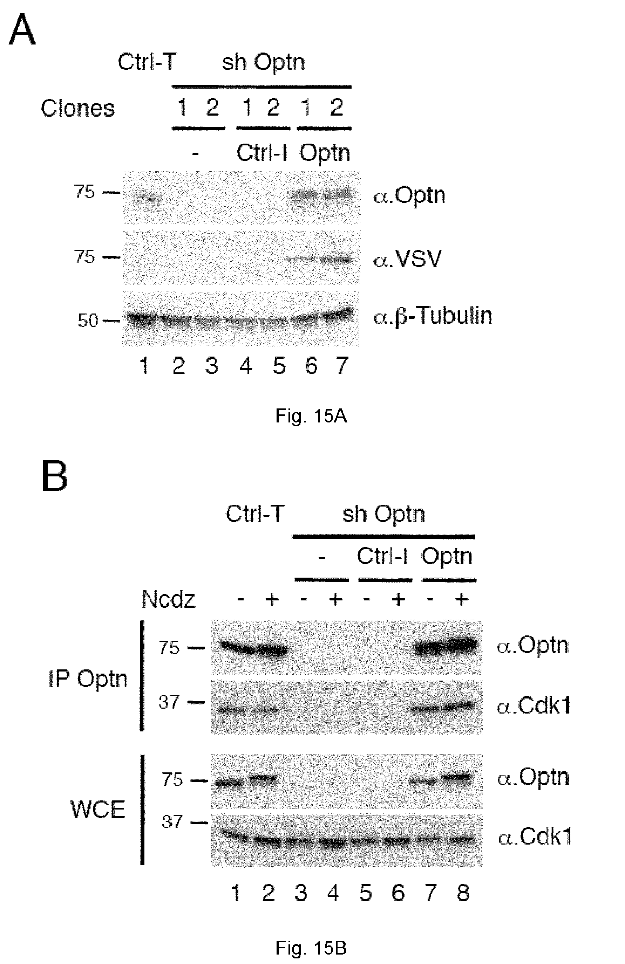

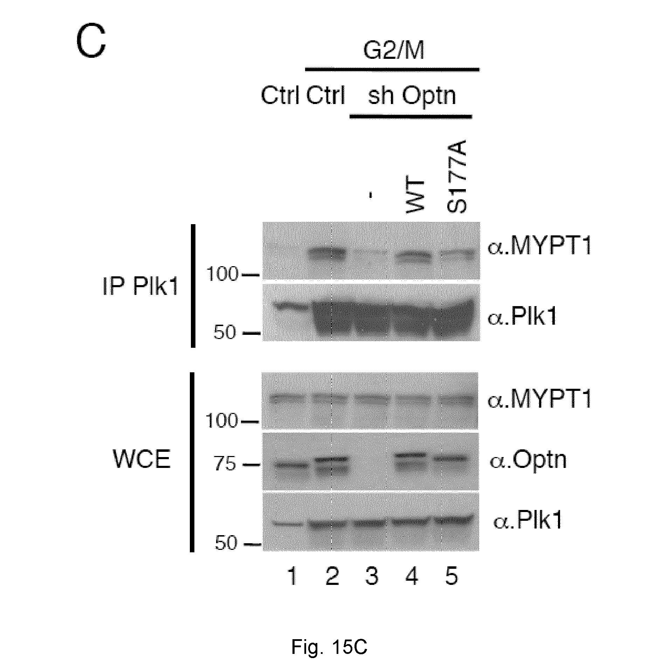

**Figure 15****: Optn interacts with Cdk1 and its phosphorylation on Ser177 is important for the interaction between MYPT1 and PIk1**
   (A) HeLa cells were stably transfected with a plasmid expressing Optn-directed shRNA or with the empty vector (Ctrl-T) and drug-resistant cells were cloned by limiting dilutions. In order to restore Optn expression, two independent Optn depleted clones (clone 1 and 2) were infected with retroviruses expressing shRNA-insensitive VSV-Optn or empty retroviruses (Ctrl-I) and selected using puromycine. Lysates were analyzed by Western blotting as indicated.
   (B) Clones of HeLa cells generated as described in panel A were treated with Nocodazole as indicated, lysed and immunoprecipitation of Optn was done with an anti-Optn antibody. Western blot analysis of immunoprecipitates and whole cell extracts (WCE) were performed using anti-Optn and anti-Cdk1 antisera.
   (C) Optn depleted HeLa cells and cells reconstituted with Optn WT and S177A were synchronized in G2/M by a double thymidine block followed by a release of 7 hours. Lysates were immunoprecipitated with an anti-PIk1 antibody. Western blots were performed using the indicated antibodies.

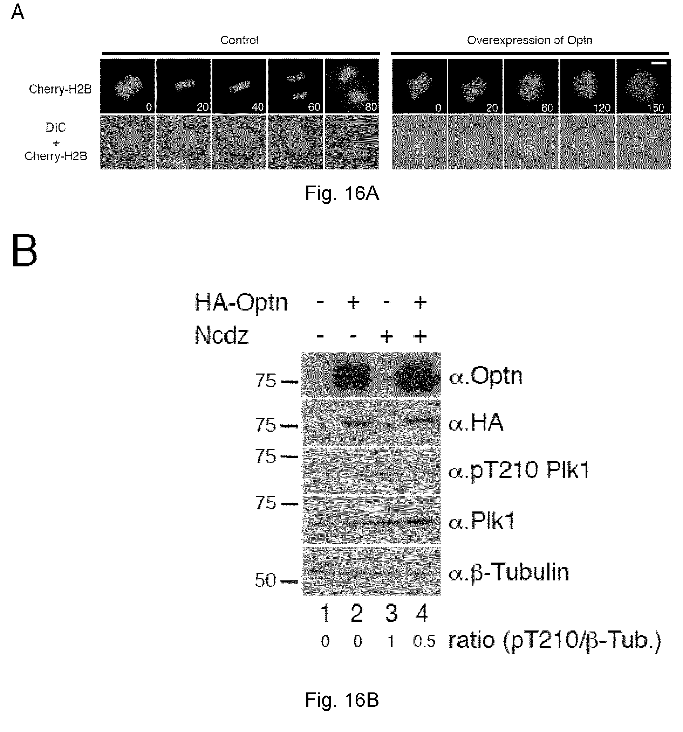

**Figure 16****: Overexpression of Optn induces mitotic arrest which correlates with inhibition of PIk1 phosphorylation.**
   (A) Live-Cell Imaging of Control and Optn-overexpressing HeLa cells. Control cells and Optn overexpressing cells were transiently transfected with a plasmid encoding Cherry-histone 2B and imaged using a Axio Observer Z1 microscope system. Images were taken at 2 min intervals. The Cherry fluorescence and the corresponding differential interference contrast images are shown. Scale bar 10 µM.
   (B) HeLa cells transfected with HA-Optn were treated with Nocodazole as indicated. The fold induction of PIk1 phosphorylation is calculated as a ratio between pT210 and β-Tubulin signals using Quantity one software.

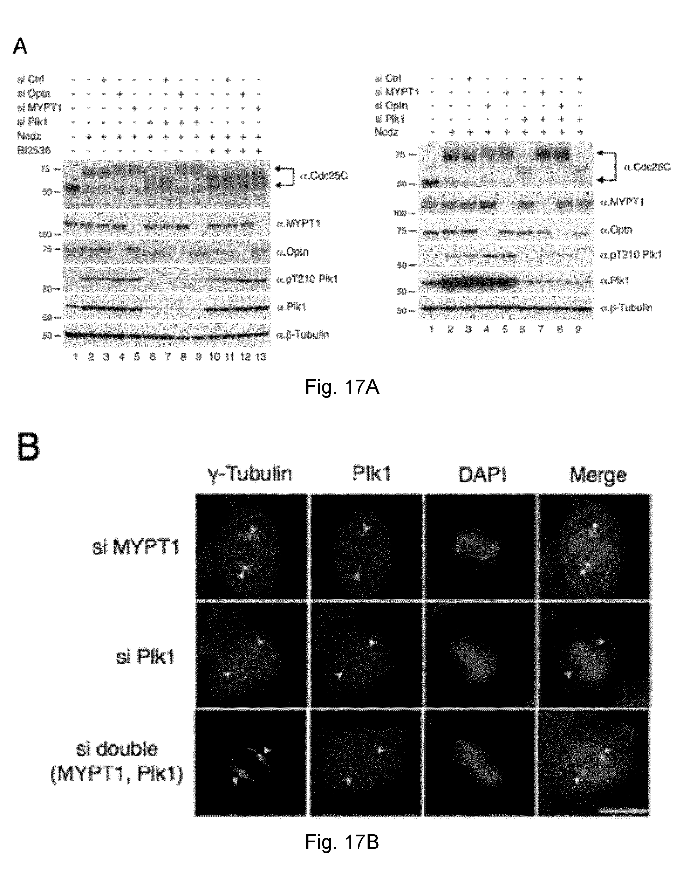

**Figure 17****: Optn and MYPT1 antagonize PIk1 mitotic targets**
   (A) **Left panel:** HeLa cells transiently transfected with the indicated siRNAs were treated with Nocodazole and BI 2536 as indicated. Depletion efficiency was determined by Western blotting with anti-MYPT1 and anti-Optn antibodies. The change in electrophoretic mobility of Cdc25C corresponding to its phosphorylation by PIk1 is detected by Western blotting with an anti-Cdc25C antibody. The position of Cdc25C and its phosphorylated isoforms are indicated by arrows. The level of expression and phosphorylation of PIk1 were monitored using respectively anti-PIk1 and anti-pT210 antibodies. Anti-β-Tubulin was used as a loading control.
      **Right panel:** A similar experiment was performed in U2OS cells.
      **These panels require a bit of explanations:**
      Consistent with previous reports (Izumi and Maller, 1993; Kumagai and Dunphy, 1992), the inventors observed a shift in the apparent molecular mass of Cdc25C from 70kDa to 100 kDa on immunoblots performed in HeLa and U2OS cells corresponding to N-terminal phosphorylation during mitosis (lanes 2). This shifted form of Cdc25C decreased when PIk1 expression was silenced (lanes 6). The effect was counteracted by the simultaneous depletion of Optn, as previously shown for MYPT1 (Yamashiro et al., 2008) (compare lanes 8, 9 to 7). In addition, the inventors observed that these double depletions restored partially the phosphorylation of remaining PIk1 as monitored by immunoblotting with the anti-pT210 (lanes 8, 9). Despite the ability to increase PIk1 phosphorylation, Optn and MYPT1 depletions could not restore Cdc25C phosphorylation when the cells were treated with BI 2536 (lanes 10-13). This result suggests that a minimum activity of PIk1 is needed to observe the antagonistic effect of Optn or MYPT1 depletion.
   (B) Double depletion of MYPT1 and PIk1 rescues the accumulation of γ-Tubulin at the centrosomes. Localization of γ-Tubulin (green), PIk1 (red) and DNA (blue) in control, MYPT1-depleted, PIk1-depleted and double depleted HeLa cells. Arrowheads indicate centrosomes. The scale bar represents 5 µm.

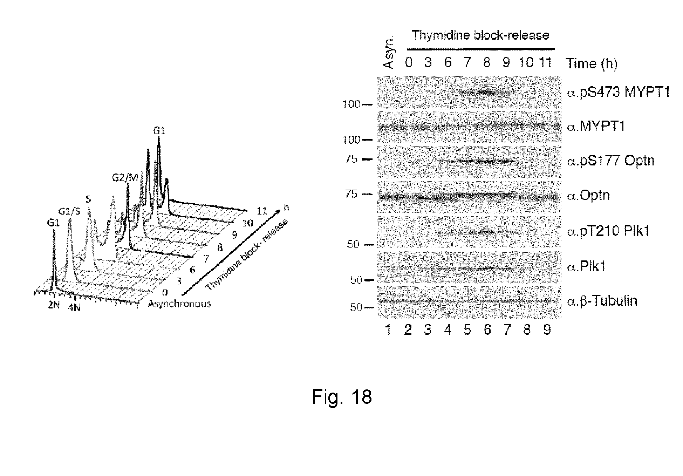

**Figure 18****: MYPT1, Optn and PIk1 are phosphorylated at the G2/M transition.**
   HeLa cells were arrested at the G1/S boundary with a double thymidine treatment and then released into fresh medium. Cells were collected at the indicated period of time and stained with propidium iodide. The cell-cycle profile was determined by flow cytometry (**left panel**) and the indicated proteins were assayed by Western blotting (**right panel**). "Asyn." indicates asynchronous cells; 2N and 4N represent DNA content.

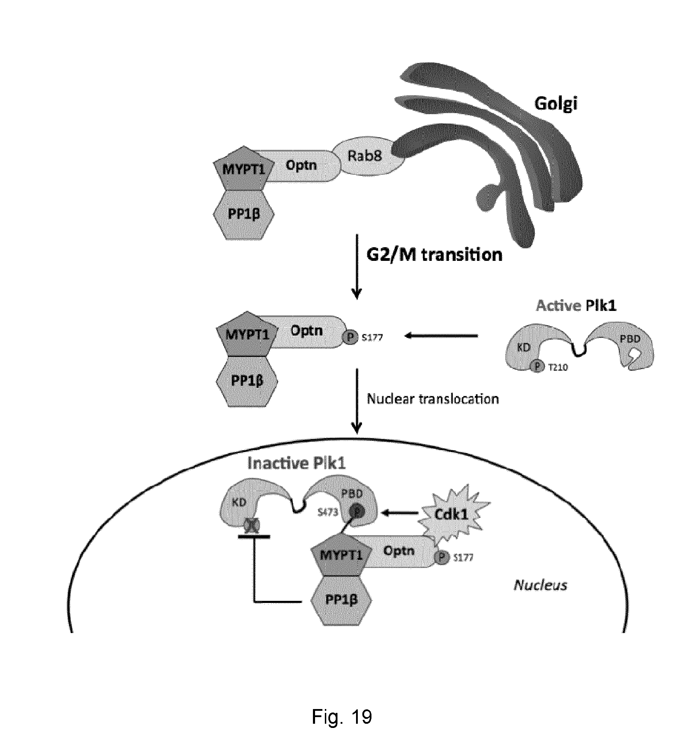

**Figure 19****: Model for the regulation of PIk1 activity.**
   PIk1 phosphorylates Optn at Ser177. This mitosis-specific phosphorylation of Optn induces its dissociation from Rab8 and its translocation from the Golgi apparatus to the nucleus. Once in the nucleus Optn allows the phosphorylation of MYPT1 by Cdk1, which generates the binding site for PIk1. Then PP1β dephosphorylates PIk1 at Thr210, its main activating site, in order to antagonize PIk1 activity.

### MOVIES:

### Movie S1:

Time-lapse recording of control HeLa cells transfected with the Cherry-Histone 2B. Pictures were taken every 2 minutes during 24h.

### Movie S2:

Time-lapse recording of Optn-depleted HeLa cells transfected with the Cherry-Histone 2B. Pictures were taken every 2 minutes during 24h.

### Movie S3:

Time-lapse recording of control HeLa cells (transfected with the empty vector). Pictures were taken every 10 minutes during 48h.

### Movies S4 and S5:

Time-lapse recording of HeLa cells stably transfected with Optn shRNA. Pictures were taken every 10 minutes.

### MATERIALS AND METHODS

### Cells, Antibodies, DNA constructs and Chemicals

HeLa, Cos-7 and HEK-293T cell lines were grown in DMEM supplemented with FBS (10%). HeLa clones stably depleted for Optn expression were selected with 200µg/ml Geneticin (G418). HeLa cell lines expressing Optn WT and mutants were selected with 1 µg/ml puromycine.

Transient transfections of HeLa cells and Cos7 with siRNA and/or plasmids were performed using ICAFectin442 (Eurogentec), JetPrime (Polyplus) or FuGENE 6 (Roche) following the manufacturer's instructions. HEK-293T cells were transfected with the Calcium Phosphate transfection method.
Stable transfection of HeLa cells with shRNA or plasmids were performed using FuGENE 6. HeLa clones were obtained by limiting dilutions. For generation of retroviral supernatants, pMSCV-puro vector (encoding different Optn constructs or control vector) was transfected into Plat-A packaging cell line using FuGENE 6. High titers of recombinant viruses were obtained 48h after transfection and used to infect.

### Constructs and siRNA

PcDNA-3-HA-tagged-Optn, VSV-tagged-Optn and -Optn D474N plasmids were previously described (Journo et al., 2009). Modified forms of Optn: HA-Optn Δ131-297, HA-Optn L150P L157P (LL/PP), HA-Optn S177A, HA-Optn S177D, HA-Optn F178R and VSV-tagged-Optn E50K were generated by site-directed mutagenesis with a PCR-based strategy. For Optn WT, Optn S177A, Optn S177D, Optn E50K and Optn D474N, RNAi insensitive constructs were created by introduction of silent mutations in the RNAi targeting sequence. The Optn constructs were subsequently transferred into the pMSCV-puro vector (Clontech) by PCR amplification at Hpa1 site. Optn cDNA fragments amplified using a pair of primers were cloned at EcoR1 and BamH1 sites of pEGFP.C2 plasmid (Clontech) in order to obtain GFP-Optn 131-297, GFP-Optn 131-253, GFP-Optn 131-210, GFP-Optn 131-170 and GFP-Optn 171-297. To construct plasmids pGST-Optn and pGST-Optn S177A, the fragments containing Optn and Optn 177A coding regions were PCR amplified and cloned into the pGEX-3X plasmid (Pharmacia). Myc-tagged-Plk1 was a kind gift of R. Goldstein. VSV-tagged-Plk1 was obtained by cloning Plk1 into pcDNA3/pT7-link-GVSV vectors at Xho1 and Xba1 sites. VSV-Plk1 K82M was obtained by site-directed mutagenesis. HA-tagged-MYPT1 and Flagtagged-PP1β were purchased from ImaGenes. GFP-Rab8 T22N and GFP-Rab8 Q67L were kindly provided by A. Zahraoui. Cherry-Histone 2B was a kind gift of S. Tajbakhsh.

Optn double-stranded siRNAs (5'GGAGACUGUUGGAAGCGAAGUdTdT), MYPT1 double-stranded siRNAs (5'GAGACAAGAAAGAUUUGCUdTdT) and β-globin double-stranded siRNAs (control, 5'GGUGAAUGUGGAAGAAGUUdTdT) were purchased from Proligo (Sigma). Plk1 double-stranded siRNAs (smart pool) was purchased from Dharmacon. The mammalian expression vector, pSuper (OligoEngine, Seattle, WA), was used for stable expression of shRNA for Optn in HeLa cells. The pSuper vector served as a non-silencing control.

### Tandem affinity purification and mass spectrometry

Tandem affinity purification using the GS-TAP cassette and analysis by mass spectrometry was performed essentially as described previously (Burckstummer et al., 2006). GS-TAP-tagged Optn expressed in HEK-293 cells was purified using rabbit immunoglobulin G (IgG) agarose and eluted by tobacco etch virus (TEV) protease cleavage. The Optn-containing complex was further purified using streptavidin agarose and eluted by boiling in SDS sample buffer. The final TAP eluate was separated on an SDS-PAGE and stained by silver staining. Protein complex composition was analyzed by LC-MSMS.

### Antibodies

The following antibodies were used: anti-VSV mAb (P5D4), anti-Myc mAb (9E10), anti-Flag M2 mAb (ref F-1804, Sigma-Aldrich), anti-HA.11 mAb (16B12, ref MMS-101 P, Covance), anti-MYPT1 rabbit polyclonal antibody (ref 07-672, Millipore), anti-PP1β rabbit polyclonal antibody (ref 07-1217, Millipore), anti-Plk1 mAb (ref sc-17783, Santa Cruz Biotechnology), anti-pT210-Plk1 mAb (ref 628902, BioLegend), anti-Cdc25C rabbit polyclonal antibody (ref sc-327, Santa Cruz Biotechnology), anti-β-Tubulin mAb (ref T4026, Sigma), anti-GFP rabbit polyclonal antibody (ref A6455, Invitrogen), anti-GFP mAb (ref 11814460001, Roche), anti-Cdk1/Cdc2 mAb (ref sc-54, Santa Cruz Biotechnology), anti-GM130 mAb (ref 610822, BD Biosciences), anti-RNA Polymerase B Subunit hRPB1 (ref PB-7C2, Euromedex), anti-NEMO rabbit polyclonal antibody (ref sc-8330, Santa Cruz Biotechnology), anti-Aurora B (ref 611082, BD Biosciences), anti-Cyclin B1 (ref sc-245; Santa Cruz Biotechnology), anti-Optn mAb D1.1 was raised in the laboratory against a Glutathione-S-transferase (GST)-Optn (aa 1-300) fusion protein. Anti-Optn rabbit polyclonal antibody was generated against a TrpE fusion protein encompassing amino acids 84-164 of human Optn. An antibody against Ser177-phosphorylated Optn (pS177) was raised in rabbit using the sequence of a KLH-conjugated phosphopeptide SGSSEDpSSFVEIR where pS indicates phosphoSerine (Eurogentec). The antibody was purified with non phosphopeptide- and phosphopeptide-conjugated agarose gel. Anti-pS473 MYPT1 mAb is a gift from S. Yamashiro (Yamashiro et al., 2008). Secondary antibodies for immunofluorescence were supplied by Molecular Probes (Alexa Fluor conjugates).

### Treatment with chemicals

Cells were incubated for 20h with 100 ng/ml Nocodazole (Sigma). For double Thymidine block and release, cells were first incubated with 2.5 mM Thymidine (Sigma) for 16h and then released for 8h. The Thymidine block was then repeated, and the second release was performed for the indicated period of time. For the G2/M border arrest, cells were treated with 9 µM RO-3306 (Calbiochem) during 20h and then released for the indicated period of time. For Plk1 kinase inhibition, cells were treated with 50nM BI 2536 (Axon MedChem) 12h before harvesting.

### Live-cell microscopy

Control cells and Optn-depleted cells transfected with Cherry-Histone 2B were imaged with the Axio Observer Z1 microscope (Zeiss) in an incubation chamber at 37°C and 5% CO₂. Images were acquired every 2 min. for 24h and analyzed with the AxioVision software 4.8.
For live-cell analyses, HeLa cells stably transfected with Optn shRNA and cells transfected with the empty vector (control), were followed by time-lapse microscopy using the Nikon Biostation IM (Nikon) at 37°C and 5% CO₂. Images were acquired every 10 minutes for 48h.

### Cell Extracts, Immunoprecipitations and Immunoblots

Lysis, immunoprecipitations and immunoblotting were performed as previously described (Lobry et al., 2007).

### Subcellular fractionations

Cytoplasmic, membrane and nuclear extracts were obtained using the Subcellular Protein Fractionation Kit, according to the manufacturer's instructions (Thermo Scientific).

### Expression and purification of recombinant proteins

pMal-c2-Cdc25C, pGST-Optn WT and pGST-Optn S177A were transformed into *E.coli* cells (BL21). Expression was induced by addition of 1 mM IPTG for 4 hours. The MBP-fusion proteins were adsorbed to amylose resin columns (New England BioLabs) and eluted following the manufacturer's instructions. The GST-fusion proteins were purified on glutathione-sepharose 4B beads (GE Healthcare).

### Kinase assay

20 mUnits of constitutive active GST-Plk1 (ref Cy-E1163, Cyclex), GST-Optn and GST-Optn S177A proteins were incubated in kinase buffer (KB) 1X (50mM Tris-HCl pH 7.5, 10mM MgCl2, 2mM EGTA, phosSTOP (Roche)) with 0.5mM ATP and [γ-³²P]ATP for 30 min at 30°C. The reactions were stopped by the addition of 2X SDS sample buffer and analysed by SDS-PAGE followed by autoradiography. For the IP-kinase assay, Plk1 was immunoprecipitated with anti-Plk1 mAb (ref sc-17783, Santa Cruz Biotechnology) as described above and incubated in the KB 1X buffer with 1 µg MBP-Cdc25C, 0.5mM ATP and [γ-³²P]ATP.

### Indirect immunofluorescence

Fixation, permeabilization and images acquisition were performed as previously described (Lobry et al., 2007).

### Cell cycle analyses

Cells were washed with PBS, fixed in cold 70% ethanol, and resuspended in PBS containing 50 µg/mL propidium iodide (Sigma) and 200 µg/mL RNase A (Sigma). Analyses were performed with a FACSCalibur flow cytometer (BD bioscience) and FlowJo v7.5.5 software.

SEQ ID N°1: Optn protein sequence












SEQ ID N°2 : Optn protein segment 131-297

LPRAEAEQEK DQLRTQVVRL QAEKADLLGI VSELQLKLNS SGSSEDSFVE IRMAEGEAEG
SVKEIKHSPG PTRTVSTGTA LSKYRSRSAD GAKNYFEHEE LTVSQLLLCL REGNQKVERL
EVALKEAKER VSDFEKKTSN RSEIETQTEG STEKENDEEK GPETVGS

SEQ ID N°3 : Optn protein segment 131-297 mutated at positions 151, 157 and 177

LPRAEAEQEK DQLRTQVVRP QAEKADPLGI VSELQLKLNS SGSSEDAFVE IRMAEGEAEG
SVKEIKHSPG PTRTVSTGTA LSKYRSRSAD GAKNYFEHEE LTVSQLLLCL REGNQKVERL
EVALKEAKER VSDFEKKTSN RSEIETQTEG STEKENDEEK GPETVGS

SEQ ID N°4 : SSGSSEDSFVEIRMA (See Figure 1B)

SEQ ID N°5 : SGGSSEDSFVEIRMT (See Figure 1B)

SEQ ID N°6 : SGGPSEDSFVEIRMA (See Figure 1B)

SEQ ID N°7 : NIASAEDSFVEIGMN (See Figure 1B)

SEQ ID N°8 : LGSFSEDSFVEIGFS (See Figure 1B)

SEQ ID N°9: Optn DNA sequence

ATGTCCCATCAACCTCTCAGCTGCCTCACTGAAAAGGAGGACAGCCCCAG
TGAAAGCACAGGAAATGGACCCCCCCACCTGGCCCACCCAAACCTGGACA
CGTTTACCCCGGAGGAGCTGCTGCAGCAGATGAAAGAGCTCCTGACCGAG
AACCACCAGCTGAAAGAAGCCATGAAGCTAAATAATCAAGCCATGAAAGG

GAGATTTGAGGAGCTTTCGGCCTGGACAGAGAAACAGAAGGAAGAACGCC
AGTTTTTTGAGATACAGAGCAAAGAAGCAAAAGAGCGTCTAATGGCCTTG
AGTCATGAGAATGAGAAATTGAAGGAAGAGCTTGGAAAACTAAAAGGGAA
ATCAGAAAGGTCATCTGAGGACCCCACTGATGACTCCAGGCTTCCCAGGG
CCGAAGCGGAGCAGGAAAAGGACCAGCTCAGGACCCAGGTGGTGAGGCTA
CAAGCAGAGAAGGCAGACCTGTTGGGCATCGTGTCTGAACTGCAGCTCAA
GCTGAACTCCAGCGGCTCCTCAGAAGATTCCTTTGTTGAAATTAGGATGG
CTGAAGGAGAAGCAGAAGGGTCAGTAAAAGAAATCAAGCATAGTCCTGGG
CCCACGAGAACAGTCTCCACTGGCACGGCATTGTCTAAATATAGGAGCAG
ATCTGCAGATGGGGCCAAGAATTACTTCGAACATGAGGAGTTAACTGTGA
GCCAGCTCCTGCTGTGCCTAAGGAAGGGAATCAGAAGGTGGAGAGACTT
GAAGTTGCACTCAAGGAGGCCAAAGAAAGAGTTTCAGATTTTGAAAAGAA
AACAAGTAATCGTTCTGAGATTGAAACCCAGACAGAGGGGAGCACAGAGA
AAGAGAATGATGAAGAGAAAGGCCCGGAGACTGTTGGAAGCGAAGTGGAA
GCACTGAACCTCCAGGTGACATCTCTGTTTAAGGAGCTTCAAGAGGCTCA
TACAAAACTCAGCAAAGCTGAGCTAATGAAGAAGAGACTTCAAGAAAAGT
GTCAGGCCCTTGAAAGGAAAAATTCTGCAATTCCATCAGAGTTGAATGAA
AAGCAAGAGCTTGTTTATACTAACAAAAAGTTAGAGCTACAAGTGGAAAG
CATGCTATCAGAAATCAAAATGGAACAGGCTAAAACAGAGGATGAAAAGT
CCAAATTAACTGTGCTACAGATGACACACAACAAGCTTCTTCAAGAACAT
AATAATGCATTGAAAACAATTGAGGAACTAACAAGAAAAGAGTCAGAAAA
AGTGGACAGGGCAGTGCTGAAGGAACTGAGTGAAAAACTGGAACTGGCAG
AGAAGGCTCTGGCTTCCAAACAGCTGCAAATGGATGAAATGAAGCAAACC
ATTGCCAAGCAGGAAGAGGACCTGGAAACCATGACCATCCTCAGGGCTCA
GATGGAAGTTTACTGTTCTGATTTTCATGCTGAAAGAGCAGCGAGAGAGA
AAATTCATGAGGAAAAGGAGCAACTGGCATTGCAGCTGGCAGTTCTGCTG
AAAGAGAATGATGCTTTCGAAGACGGAGGCAGGCAGTCCTTGATGGAGAT
GCAGAGTCGTCATGGGGCGAGAACAAGTGACTCTGACCAGCAGGCTTACC
TTGTTCAAAGAGGAGCTGAGGACAGGGACTGGCGGCAACAGCGGAATATT
CCGATTCATTCCTGCCCCAAGTGTGGAGAGGTTCTGCCTGACATAGACAC
GTTACAGATTCACGTGATGGATTGCATCATTTAA

SEQ ID N°10 : Optn protein segment 131-297 mutated at position 177

LPRAEAEQEK DQLRTQVVRL QAEKADLLGI VSELQLKLNS SGSSEDAFVE IRMAEGEAEG
SVKEIKHSPG PTRTVSTGTA LSKYRSRSAD GAKNYFEHEE LTVSQLLLCL REGNQKVERL
EVALKEAKER VSDFEKKTSN RSEIETQTEG STEKENDEEK GPETVGS

### RESULTS

### Optn is phosphorylated by Plk1 during mitosis

The inventors observed that Optn immunoprecipitated from HeLa cells arrested in prometaphase by Nocodazole treatment underwent a shift to a higher apparent molecular weight, as indicated by SDS-PAGE (Figure 1A). This slow migrating form of Optn disappeared following lambda phosphatase treatment, indicating that this shift was phosphorylation-dependent (compare lane 8 to lanes 2, 4, 6). Based on these findings, the inventors sought to identify mitosis-specific phosphorylation sites on Optn. By analyzing the Optn protein sequence, the inventors identified a sequence surrounding Ser177 that matches the consensus phosphorylation sequence for PIk1, D/E-X-S/T-Φ-X-D/E (X, any amino acid; Φ, a hydrophobic amino acid) (Nakajima et al., 2003) and that is conserved among known Optn homologs (Figure 1 B). To determine whether Plk1 could phosphorylate Optn on Ser177, the inventors performed *in vitro* kinase assays using GST-Optn as a substrate and a constitutively active human full length Plk1 as a source of kinase activity (Figure 1C). The inventors observed that active Plk1 could phosphorylate wild type Optn, but not a mutant form where the putative acceptor serine residue was replaced by alanine (S177A) or when BI 2536, a selective Plk1 inhibitor, was added to the kinase reaction. Consistently, a shift in Optn migration was observed in HeLa cells expressing wild type PIk1, but not a kinase dead form of Plk1 (Plk1 K82M) (Figure 1D, compare lane 2 to 3) and was prevented when Optn was mutated at Ser177 (S177A:lane 5) or at Phe178 (F178R; Figure 8A). To further investigate the phosphorylation of Optn at Ser177, the inventors generated a phospho-specific antibody (pS177) against the peptide epitope surrounding Ser177. This antibody detected wild type Optn, but not S177A, when co-expressed with Plk1 (Figure 1D; compare lane 2 to 5). The pS177 antibody also detected endogenously-phosphorylated Optn in HeLa cells arrested in mitosis with Nocodazole (Figure 1E, lane 3) or Taxol (data not shown), but not in cells depleted for Plk1 by siRNA or treated with BI 2536 (lanes 4, 5). Next, the inventors used this phosphospecific antibody to identify the kinetics of Optn phosphorylation on Ser177 in HeLa cells that were synchronized at the G1-S boundary by a double thymidine block and release (Figure 1 F). Expression of Plk1 increased 4 hours after the release, while phosphorylation of both Optn on Ser177 (pS177) and Plk1 on Thr210 (p210) were detected later in the G2 phase (6 hours after release), peaked at the onset of cyclin B degradation (8-10 hours) and decreased dramatically at the end of mitosis (12 hours). The timing of Optn phosphorylation also paralleled that of Plk1 phosphorylation when HeLa cells were released from a Cdk1-inhibitor RO-3306 that synchronizes the cells in late G2 (Vassilev et al., 2006) (Figure 8B). Altogether, these data demonstrate that Plk1 phosphorylates Optn during mitosis.

### Optn phosphorylation induces its translocation into the nucleus by preventing its interaction with the Golgi-resident protein Rab8

The inventors and others have previously observed that Optn predominantly localizes to the Golgi apparatus in unsynchronized cells ((De Marco etal., 2006; Rezaie et al., 2002; Schwamborn et al., 2000), Figure 9A) and that the [aa 131-297] region of Optn is necessary (Figure 9B) and sufficient (Figure 9C) to promote Golgi localization. Since this region encompasses the Plk1 phosphorylation site, the inventors hypothesized that this phosphorylation could also affect the subcellular localization of Optn. Accordingly, the inventors observed that Optn no longer localized to the Golgi region and was mainly found into the nucleus (68%, n=50) when coexpressed with wild type PIk1, but not with the K82M mutant (8%, n=50) (Figure 2A). Similar experiments performed with the phosphodeficient mutant Optn S177A showed that this mutated form remained perinuclear when Plk1 was co-expressed (4% nuclear localization, n=50). In contrast, 48% (n=50) of the phosphomimetic mutant, Optn S177D, displayed a nuclear localization independently of Plk1 expression, suggesting that Ser177 phosphorylation could be responsible for Optn nuclear relocalization. To further investigate the intracellular distribution of Optn, HeLa cells were synchronized at the G2/M phase (Figure 2B) and then fractionated into three fractions: a cytoplasmic (C), a membrane (M) and a nuclear (N) fraction. In asynchronous cells, Optn was mainly found in the cytosolic fraction, while it accumulated in the nucleus with its phosphorylated form in G2/M synchronized cells. No cross-contaminations were found in the different cell fractions when analyzed for the presence of the cytosolic NEMO protein and the nuclear RPB1 protein. Consistent with these data, immunofluorescence experiments confirmed that Optn and its phosphorylated product were exclusively in the nucleus of cells synchronized at the G2/M border (Figure 2C and Figure 10A) and paralleled Aurora B localization indicating that the nuclear accumulation of Optn occurs at the end of the G2 phase (Delaval et al., 2004). The specificity of the pS177 antibody was confirmed by the absence of staining in Optn-stably depleted cells (Figure 10B). As expected, Optn remained mainly cytoplasmic (perinuclear localization in 86% of cells, n=50) when the arrested-mitotic cells were further treated with the selective Plk1 inhibitor BI 2536, while this treatment had no effect on Plk1 localization (Figure 2C, right panel).

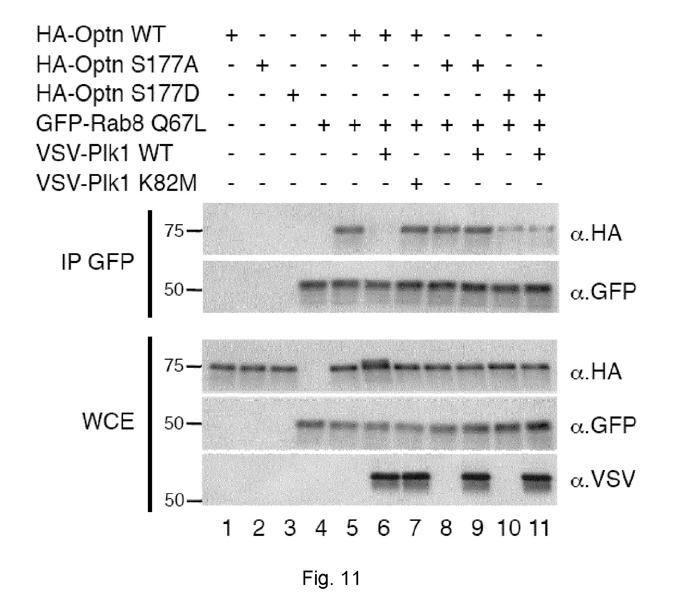

Similar results were obtained when assessing the localization of stably-transfected GFP-tagged Optn after synchronization in mitosis (Figure 10C). The [aa 141-209] region of Optn, that encompasses the S177 phosphorylation site has been previously shown to mediate the association of Optn with the GTP-bound form of Rab8 (Rab8 Q67L), suggesting that the Plk1-dependent phosphorylation of Optn could modulate this interaction. Indeed, co-immunoprecipitation experiments performed in HeLa cells indicated that Optn phosphorylation by Plk1 disrupted its binding with Rab8 Q67L, while the kinase dead mutant of Plk1 did not affect this interaction (Figure 3A, compare lanes 5-7). Consistently, the inventors found that Optn S177A interacted with Rab8 Q67L and that this interaction was not abolished upon Plk1 expression, whereas the interaction of Optn S177D with Rab8 Q67L was strongly decreased independently of Plk1 expression (Figure 3A, lanes 8-11). The same results were also obtained in HEK-293T cells (Figure 11). Next, the localization of phosphodeficient and phosphomimetic mutants of Optn was compared to the localization of Rab8 Q67L in the presence or absence of Plk1. Optn WT and Optn S177A alone were diffusely perinuclear (see Figure 2A), while both Optn forms colocalized in tight perinuclear spots when coexpressed with Rab8 Q67L (70% of colocalization, n=30), but not with GDP-bound form Rab8 T22N (Figure 3B and S2D). In contrast, Rab8 Q67L expression did not affect the localization of Optn S177D. To further support these observations, the inventors examined the effect of Plk1. As expected, Plk1 WT, but not the kinase-dead form, impaired the colocalization of Optn and Rab8 Q67L and induced the nuclear localization of Optn (73%, n=30) (Figure 3B). However, Plk1 was unable to disrupt the colocalization of Optn S177A and Rab8 Q67L. These results suggest that upon phosphorylation by Plk1, Optn dissociates from Rab8 and consequently translocates into the nucleus.

### Identification of putative Optn interacting partners by mass spectrometric-based proteomics

To identify novel partners of Optn that could account for a mitotic function of Optn, the inventors generated stable HEK-293 cells lines that express a GS-TAP-tagged version of Optn and purified the Optn complex by tandem affinity purification (Burckstummer et al., 2006). Mass spectrometry analysis presented in Figure 4A identified Optn and the already described Optn-interactor Myosin VI (Sahlender et al., 2005). Interestingly, amongst the putative novel interactors of Optn listed in Table 1, the two main subunits of the myosin phosphatase holoenzyme (MP) were identified. MP is composed of the type 1 protein phosphatase catalytic subunit (PP1cβ) and the myosin phosphatase targeting subunit (MYPT1). Two known interactors of MYPT1, myosin phosphatase Rho interacting protein (M-RIP) and prostate apoptosis response 4 protein (PAR4) (Vetterkind et al., 2010) were also identified in our proteomic approach. MP was recently shown to regulate mitotic progression by antagonizing Plk1 essential functions during mitosis (Yamashiro et al., 2008). The inventors therefore focused their attention on this phosphatase complex since its interaction with Optn may explain its mitotic function.

### Table 1 : List of the interacting proteins identified by liquid chromatography tandem mass spectrometry (LC-MSMS) from a tandem affinity purification (TAP) of GS-tagged Optn.

The number of uniquely-identified peptides, tandem mass spectra and sequence coverage are given for each protein.

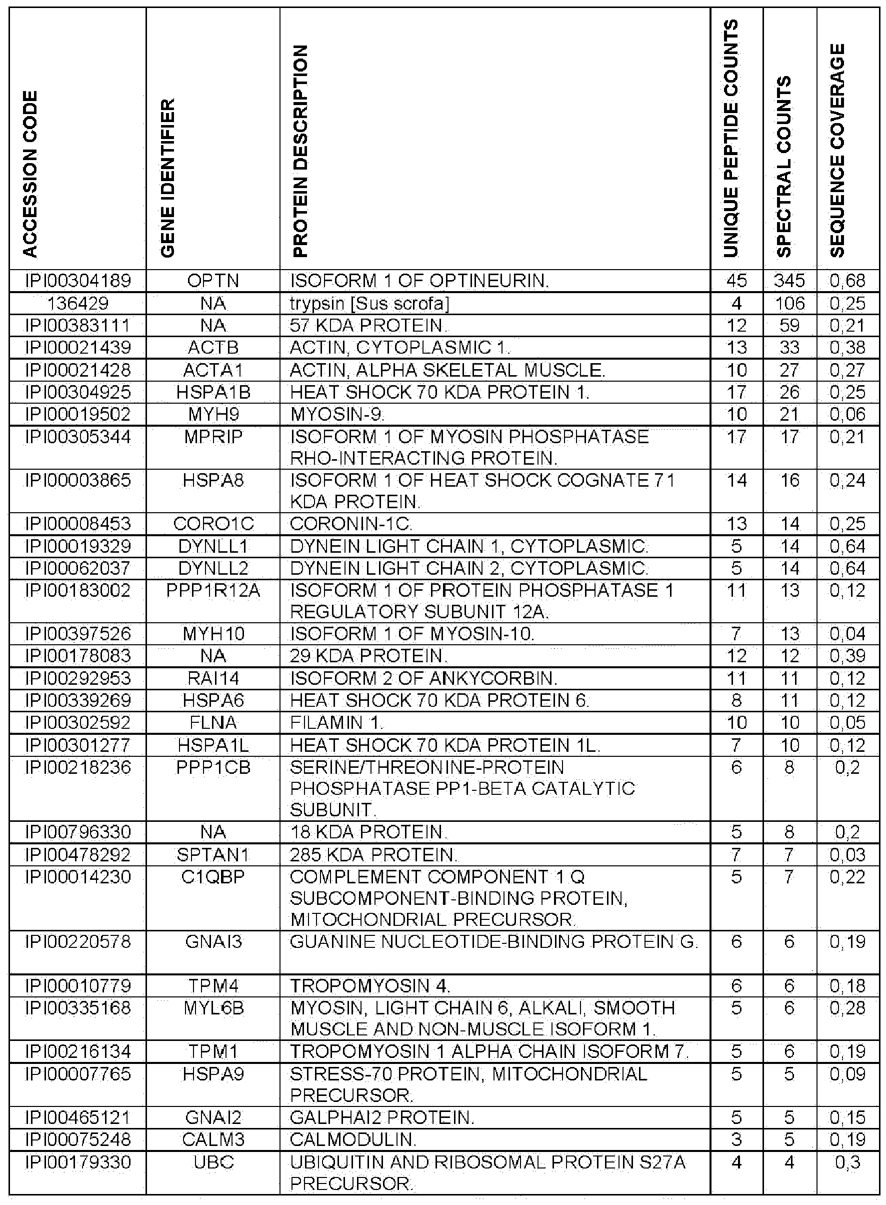

| | | | | | |
|---|---|---|---|---|---|
| IP I00796776 | NA | 60 KDA PROTEIN | 4 | 4 | 0,07 |
| IP I00300096 | RAB35 | RAS-RELATED PROTEIN RAB-35 | 3 | 3 | 0,16 |
| IP I00014424 | EEF1A2 | ELONGATION FACTOR 1-ALPHA 2 | 3 | 3 | 0,06 |
| IP I00033494 | NA | MYOSIN REGULATORY LIGHT CHAIN | 3 | 3 | 0,19 |
| IP I00376344 | MYO1B | ISOFORM 1 OF MYOSIN IB | 3 | 3 | 0,03 |
| IP I00000874 | PRDX1 | PEROXIREDOXIN-1. | 2 | 2 | 0,11 |
| IP I00003348 | GNB2 | GUANINE NUCLEOTIDE-BINDING PROTEIN G(I)/G(S)/G(T) SUBUNIT BETA 2 | 2 | 2 | 0,06 |
| IP I00010438 | SNAP23 | ISOFORM SNAP-23A OF SYNAPTOSOMAL-ASSOCIATED PROTEIN 23 | 2 | 2 | 0,11 |
| IP I00001871 | PAWR | PRKC APOPTOSIS WT1 REGULATOR PROTEIN | 2 | 2 | 0,09 |
| IP I00005614 | SPTBN1 | ISOFORM LONG OF SPECTRIN BETA CHAIN, BRAIN 1 | 2 | 2 | 0,01 |
| 56160532 | NA | E1 B 55K [Human adenovirus type 5] | 2 | 2 | 0,01 |
| IP I00795269 | NA | 11 KDA PROTEIN | 2 | 2 | 0,22 |
| IP I00008455 | MY06 | ISOFORM 2 OF MYOSIN-6 | 2 | 2 | 0,02 |
| IP I00028931 | DSG2 | DESMOGLEIN 2 PREPROPROTEIN | 2 | 2 | 0,02 |
| IP I00013808 | ACTN4 | ALPHA-ACTININ-4 | 2 | 2 | 0,02 |

### Validation of the interaction between Optn and the Myosin phosphatase complex

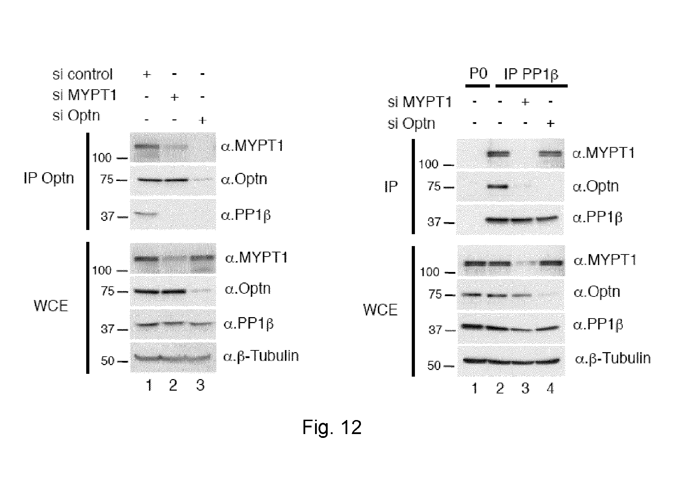

To validate the interaction of Optn with MYPT1 and PP1β the inventors performed co-immunoprecipitation experiments in HEK-293T cells (Figure 4B). Both MYPT1 and Optn were detected in PP1β immunoprecipitates when the three proteins were co-expressed, but not when MYPT1 was not expressed (Figure 4B, lanes 4 and 5). As shown in Figure 4C, endogenous MYPT1 and PP1β were detected in the anti-Optn immunoprecipitates. As expected, these detections decreased when Optn expression was silenced by siRNA and the interaction between Optn and PP1β was reduced when MYPT1 was depleted by siRNA indicating that association of Optn with the MP complex was most likely mediated by MYPT1. The reverse experiments (i.e. immunoprecipitation of PP1β) confirmed that the interaction between PP1β and Optn was dependent on MYPT1 expression (Figure 4C, compare lanes 5, 6 and 7). The same results were obtained in HEK-293T cells (Figure 12). Further experiments indicated that immunoprecipitation of endogenous and exogenous MYPT1 led to the recovery of Optn, but not of Optn Δ131-297 suggesting that MYPT1 binds the [131-297] region of Optn (Figure 4D, lanes 1, 5 and 7). MYPT1 contains a C-terminal Leucine Zipper motif (LZ), which is responsible for LZ-LZ-mediated interaction with other partners such as PAR4 (Vetterkind et al., 2010). As the MYPT1-interacting region of Optn encompasses a putative LZ motif, the inventors speculated that binding of these two proteins could be mediated by a LZ-LZ interaction. Indeed, replacement of two leucine residues (L150P, L157P) was sufficient to severely reduce the interaction between Optn and MYPT1 (Figure 4D, lane 6). Co-immunoprecipitation experiments performed with GFP-tagged constructs containing N- and C-terminal deletions of the [131-297] region of Optn indicated that the [131-170] region of Optn containing the putative LZ was sufficient for the interaction with MYPT1 (Figure 4E).

### Optn is involved in the negative regulation of Plk1

The ability of MYPT1 to antagonize Plk1 function (Yamashiro et al., 2008) raised the possibility that Optn could participates in the regulation of Plk1 activity through its association with MYPT1. To test this hypothesis, the inventors performed RNA interference experiments in HeLa cells that were arrested in mitosis upon treatment with Nocodazole (Figure 5A). As expected, both Plk1 expression and phosphorylation on Thr210 were increased in Nocodazole-arrested cells compared to unsynchronized cells (Figre 5A, lanes 1 and 2) and knockdown of MYPT1 expression resulted in a 2.9 fold enhancement of this phosphorylation (lane 4). Interestingly, the inventors observed a similar fold increase of Plk1 phosphorylation in Optn-depleted cells (lane 5), which was reversed following expression of increasing amounts of an RNAi-insensitive Optn construct, but not of the empty vector alone (Figure 5A, compare lanes 6-8 to 9-11), suggesting that Optn is involved in the regulation of Plk1 phosphorylation. Consistently, Plk1 immunoprecipitated from MYPT1- and Optn-depleted mitotic cells showed a similar increase in kinase activity (>2 fold) (Figure 5B, compare lanes 4-5 to 2-3), as evidenced by Plk1 auto-phosphorylation or phosphorylation of Cdc25C phosphatase, a known substrate of Plk1 (Archambault and Glover, 2009). As expected, Plk1 kinase activity was abrogated when Plk1 was immunoprecipitated from cells treated with BI 2536 (Figure 5B, lane 6). These results demonstrate that, like MYPT1, Optn is involved in the negative regulation of Plk1 during mitosis. To determine the kinetics of Plk1 Thr210 phosphorylation during the G2/M phase in the absence of Optn, HeLa cells stably depleted for Optn were synchrosnized with a double thymidine block protocol and released during 12 hours (Figure 5C). In these cells, Plk1 phosphorylation level was increased by 2- to 2.6-fold from 6 to 10 hours post release compared to control cells. The inventors next performed immunofluorescence microscopy using double labeling with anti-Plk1 and anti-pT210 antibodies to determine the stage of mitosis at which Plk1 phosphorylation was increased in Optn-depleted cells. The inventors observed that Optn depletion enhanced Plk1 Thr210 phosphorylation at all the mitotic phases from prophase to cytokinesis, in coherence with the kinetic of Plk1 phosphorylation (Figure 5D). Quantitative analysis revealed that the mean ratio of pT210/Plk1 fluorescent intensity in Optn-depleted cells was increased from 1.5 to 1.8 compared to control cells (Figure 13).

### Optn is essential for MYPT1 phosphorylation during mitosis

To establish the function of Optn in MYPT1-mediated regulation of Plk1 activity, the inventors evaluated whether Optn was connected to the previously described mitotic complex between MYPT1 and Plk1 (Yamashiro et al., 2008). They observed that Optn was recruited to this complex through its interaction with MYPT1 (Figure 14B). More interestingly, they observed that silencing Optn in HeLa or HEK-293T cells impaired the interaction between endogenous Plk1 and MYPT1 during mitosis (Figure 6A and 14A). Since it has been previously shown that Cdk1 creates the docking site on MYPT1 for Plk1 (Yamashiro et al., 2008), this observation suggested that Optn could be involved in Cdk1-mediated MYPT1 phosphorylation. In order to assess this hypothesis, the inventors generated stably-transfected HeLa cell lines under limiting dilution conditions in which endogenous Optn was depleted by short hairpin RNA. The two clones isolated exhibited almost undetectable levels of Optn expression (Figure 15A). The inventors also stably restored Optn expression in the clones by transducing Optn-depleted cells with a retroviral vector encoding shRNA-insensitive Optn or the empty retroviral plasmid as negative control. In agreement with the inventors' hypothesis, MYPT1 phosphorylation at Ser473, the Cdk1 preferential site (Yamashiro et al., 2008), was almost abolished in mitotic HeLa cells depleted for Optn compared to parental HeLa cells (Figure 6B, compare lane 8 to lanes 9, 10). Phosphorylation was restored in depleted cells stably re-expressing Optn but not in those transduced with the empty vector (Figure 6B, lanes 11-14). As expected, levels of Plk1 phosphorylation at Thr210 and activity (measured by *in vitro* kinase assay) were inversely correlated to MYPT1 phosphorylation levels at Ser473 (Figure 6B). In addition, Optn was constitutively associated with Cdk1 and this interaction was not modified upon Nocodazole treatment (Figure 15B). Taken together, these results strongly suggest that Optn stimulates Cdk1-induced MYPT1 phosphorylation leading to Plk1 inactivation during mitosis.

### Role of Serine 177 phosphorylation in the regulation of Plk1 activity during mitosis and in mitotic progression

The inventors monitored Plk1 and MYPT1 phosphorylation in HeLa cells depleted for Optn and reconstituted with shRNA-insensitive mutants (Figure 6C). The inventors found that ectopic expression of wild type Optn and S177D mutant could rescue the mitotic phosphorylation of MYPT1 and the regulation of Plk1 phosphorylation, in contrast to Optn S177A (Figure 6C, lanes 10-12). In agreement with these results, the interaction between MYPT1 and Plk1 (which is dependent on MYPT1 phosphorylation) was also reduced in HeLa cells expressing Optn S177A (Figure 15C). As predicted by results obtained by Plk1 overexpression (Figure 2A), this mutant was unable to translocate into the nucleus of G2/M synchronized cells, while Optn S177D was constitutively localized in the nucleus in 65% of the cells (n=50) (Figure 6D). In contrast to Optn S177A, the mutation of the ubiquitin-binding domain of Optn (D474N) and the main mutation associated with primary open-angle glaucoma (E50K) did not interfere with the ability to rescue the defect of Plk1 regulation caused by depletion of Optn (Figure 6C, lanes 13 and 14). Altogether, these results demonstrate that the translocation of Optn into the nucleus promotes the phosphorylation of MYPT1 and its interaction with Plk1, which then enables the inactivation of Plk1. Interestingly, two or more nuclei were observed by immunofluorescence microscopy in more than 10% of Optn-depleted HeLa cells in contrast to control HeLa cells that only show 2% of multinucleated cells (Figure 7A). The inventors further monitored the number of multinucleated cells in Optn-depleted cells stably reconstituted with Optn mutants. They observed that expression of Optn WT and S177D mutant rescued the multinucleation defect, while S177A did not. These results suggest that the translocation of Optn into the nucleus is required for the regulation of Plk1 activity and consequently for mitotic progression.

### Optn is required for mitotic progression

To characterize these mitotic defects, the inventors performed live-cell imaging of Cherry-Histone 2B in HeLa cells stably depleted for Optn or overexpressing Optn. Control cells underwent the metaphase-anaphase transition between 40 and 60 min after chromosome condensation (Figure 7B and Movie S1). Compared to these cells, 25% of Optn depleted cells showed a delay (between 60 and 180 min) in the metaphase-anaphase transition and failed to achieve their division leading eventually to multinucleations. As exemplified on movie S2, it was also frequently observed that chromosomes did not congress to the metaphase plate and during mitotic exit, DNA bridges were visualized suggesting the presence of chromosomal segregation defects. Interestingly, overexpression of Optn in HeLa cells resulted in mitotic arrest and cell death for 15% of mitotic cells in correlation with a 2-fold decrease in Plk1 phosphorylation during mitosis (Figures 16A and 16B). Indeed, these cells failed to enter into metaphase even after 2 hours observation, as depicted in Figure 16A. Moreover, time-lapse microscopy analysis indicated that 10% of Optn depleted cells displayed defects during late cytokinesis, including failure to complete abscission, increased duration of mitosis and frequent multinucleation due to intercellular bridge instability (Movies S4 and S5), while control HeLa cells progressed through mitosis from prophase to telophase with normal kinetics (Movie S3). The multinucleated Optn depleted cells either divided or failed to enter the next cell division and eventually died.

### Optn antagonizes Plk1 functions

To determine the biological significance of the regulation of Plk1 activity by Optn, the inventors assessed the consequences of Optn depletion on different mitotic effects of Plk1 such as Cdc25C phosphorylation (which promotes entry in mitosis) and□ γ-Tubulin recruitment to mitotic centrosomes (which contributes to centrosome maturation). Using an anti-Cdc25C antibody, the inventors observed that double depletion of Optn and Plk1 restored Cdc25C phosphorylation, which was impaired upon silencing of Plk1 (Figure 17A). Plk1 inactivation in mammalian cells has been shown to block the recruitment of γ-Tubulin at the centrosomes (Petronczki et al., 2008). The inventors therefore analyzed whether double depletion of Optn and Plk1 restores the detection of γ-Tubulin at the centrosomes, as it has been shown for the double Plk1 and MYPT1 depletion (Yamashiro et al., 2008). In cells transfected with a control siRNA, γ-Tubulin was present at the centrosomes where it colocalized with Plk1, while its accumulation at the centrosomes was greatly reduced in Plk1-depleted cells, confirming previous studies (Petronczki et al., 2008) (Figure 7C and 17B). In contrast, double depletions of Optn and Plk1 as well as MYPT1 and Plk1 restored γ-Tubulin accumulation at the centrosomes to a similar level than the control. The inventors did not observe any effect of Optn or MYPT1 single depletion on γ-Tubulin staining at the centrosomes. Quantitative measurement confirmed the above conclusion (Figure 7C). These results further support the conclusion that Optn antagonizes Plk1 functions in mitotic progression.

### DISCUSSION

Through their study, the inventors provided evidence that Optn plays an important role in the regulation of Plk1 activity during mitosis, a function that has escaped attention so far. The first step of this regulation depends on the phosphorylation of a conserved residue (Ser177) of Optn by Plk1 that promotes detachment of Optn from the Golgi-localized Rab8 small GTPase and its translocation into the nucleus (schematized in Figure 19). In the second step, nuclear Optn promotes phosphorylation of its interacting partner MYPT1 on the preferential site for Cdk1 (Ser473). This phosphorylation creates a specific binding motif for the polo box domain of Plk1, thereby allowing PP1β, the catalytic subunit of the myosin phosphatase complex to dephosphorylate Plk1 at Thr210 (Yamashiro et al., 2008). The inventors' work highlights the role of Optn in the functional interplay between Cdk1 and MYPT1 to regulate Plk1 activity. The absence of Optn homologs in invertebrates such as *Drosophila* and *Caenorhabditis elegans* and the lack of conservation of the MYPT1 phosphorylation site responsible for Plk1 binding in these species (Yamashiro et al., 2008) suggest that this Optn-mediated regulatory mechanism is not conserved in flies and worms.
First, this study details the molecular mechanism of the temporal regulation of Plk1 activity. Similar kinetic patterns of Optn, MYPT1 and Plk1 phosphorylation suggest that all these phosphorylation events transiently occur during a short period of time at the G2/M transition (Figure 18). Using Optn phosphodeficient mutants, the inventors demonstrated that Optn phosphorylation on Ser177 is required for the induction of MYPT1 phosphorylation, although the MYPT1/PP1β complex does not appear to be involved in the dephosphorylation Optn (data not shown). Second, the inventors' work further demonstrates that Optn also orchestrates spatial regulation of Plk1. Indeed, translocation of Optn from the Golgi apparatus into the nucleus triggered by Plk1-mediated phosphorylation constitutes a prerequisite for the downregulation of Plk1 activity by Optn. Plk1 has already been demonstrated to control the localization of some of its substrates: Plk1 phosphorylates the centrosome-associated protein Cep55 and prevents its recruitment to the central spindle during anaphase, therefore ensuring efficient abscission (Bastos and Barr, 2010). In addition to Optn, other proteins have been shown to dissociate from the Golgi apparatus upon phosphorylation. For example, phosphorylation of the peripheral Golgi protein Nir2 by Cdk1 induces localization of Nir2 to the cleavage furrow and midbody and hence ensures the completion of cytokinesis (Litvak et al., 2004). Strikingly, Optn depletion induces cytokinesis defects in agreement with other studies suggesting that Plk1 specifically controls the onset of cytokinesis during late mitosis. Plk1 plays a key role in triggering the initiation of cytokinesis in human cells by promoting interaction between the Rho guanine nucleotide exchange factor (GEF) Ect2 and the RhoGAP HsCyk-4. Formation of this complex is required for the activation of RhoA (the upstream regulator of the contractile ring) at the equatorial cortex during anaphase and for ingression of the cleavage furrow (Brennan et al., 2007; Petronczki et al., 2007; Santamaria et al., 2007). Plk1 is also required for stable localization of a number of other spindle components and cytokinetic regulators. In contrast, Optn does not localize to the midbody during cytokinesis (data not shown), suggesting that it may not have a direct role in cytokinesis. On the other hand, the inventors' proteomic analysis identified Optn-interacting proteins such as dynein, Rab35 and myosin VI, involved in cytokinesis (Arden et al., 2007; Campbell et al., 1998; Karki et al., 1998; Kouranti et al., 2006). Further work is needed to determine whether these proteins are downstream or upstream targets of the Optn/MYPT1/Plk1 pathway. In the last few years, Optn has been suggested to be involved in a variety of functions (Chalasani et al., 2009). Interestingly, following identification of three Optn partners by two-hybrid screens (Rab8, Myosin VI and Hungtingtin), a series of publications (Chalasani et al., 2009) have focused on the role of Optn in Golgi organisation, exocytosis and more general membrane-trafficking events. These studies suggest that Optn recruits Hungtingtin to Rab8, and therefore to the Golgi apparatus, hypothesizing a possible role for Optn in connecting the Golgi apparatus to the microtubule network (Hattula and Peranen, 2000). Optn have also been involved in other pathways including protection against oxidative stress-induced apoptosis, regulation of agonist-stimulated Group I metabotropic glutamate receptors (mGluR1) signaling, cyst formation, regulation of endocytic trafficking of transferrin receptor and more recently selective autophagy of *Salmonella enterica* (Chalasani et al., 2009; Nagabhushana et al., 2010; Wild et al., 2011). Despite structural homology with NEMO, the core element of the NF-kB pathway, and although Optn can modulate NF-κB activation in some circumstances, the inventors previously demonstrated that Optn is not part of the IKK complex (Schwamborn et al., 2000). NEMO contains an ubiquitin-binding domain (UBD) required for NF-κB activation (Israel, 2006). Results presented in this study demonstrate that the function of Optn in the regulation of Plk1 activity is independent of its UBD. In contrast, the inventors showed that the phosphorylation of Optn at Ser177 plays a pivotal role in mitotic progression related to Plk1 activity. Interestingly, it has been shown recently that phosphorylation of the same site by TBK1 is also critical for the clearance of *Salmonella* by autophagy (Wild et al., 2011). It has been reported that Optn translocates from the Golgi to the nucleus in response to H₂O₂ (De Marco et al., 2006). While this latter situation parallels what occurs during mitosis, the mechanism leading to Optn nuclear transport stimulated by H₂O₂, is unlikely to be related to Plk1 activation, neither to Ser177 phosphorylation (data not shown). The mechanism underlying the nuclear transport of Optn is still unclear since it lacks obvious nuclear localization sequences.
Optn has been linked to different pathologies since mutations in the *Optn* gene (the most frequent being E50K) have been associated with primary open-angle glaucoma (POAG) (Chalasani et al., 2009) and amyotrophic lateral sclerosis (ALS) (Maruyama et al., 2010). The molecular mechanism by which Optn mutations lead to these pathologies has not been established so far. The inventors' results indicate that neither the E50K mutation, nor D474N (mutation of the UBD) impairs the ability of Optn to regulate Plk1. This suggests that pathogenic mutations of Optn are not directly related to the mitotic function of Optn the inventors describe in their study, and are either associated with other yet to be characterized functions, or are gain of function mutations. In contrast, the negative feedback mechanism of Plk1 activation by Optn is potentially relevant for models of tumorigenesis. Plk1 is a key regulator of mitosis and, not unexpectedly, its upregulation has been linked to many cancers. The inventors' study indicates that Optn is a negative regulator of Plk1, suggesting that its downregulation might be linked to carcinogenesis. In support of this hypothesis, downregulation of Optn in invasive urothelial carcinoma has been reported (Li et al., 2008). Further work is required to determine whether altered expression of Optn might account for uncontrolled Plk1 activity and tumorigenesis.

### CONCLUSION

Plk1 activation is required for progression through mitotic entry to cytokinesis. Experiments performed by the inventors allowed showing that at mitotic entry, Plk1 phosphorylates Optineurin (Optn) at serine 177 and that this dissociates Optn from the Golgi-localized GTPase Rab8, inducing its translocation into the nucleus. Mass spectrometry analysis revealed that Optn is associated with a Myosin Phosphatase complex (MP), which antagonizes the mitotic function of Plk1. Moreover inventors' data indicate that Optn functionally connects this complex to Plk1 by promoting phosphorylation of the myosin phosphatase targeting subunit 1 (MYPT1). Accordingly, silencing Optn expression increases Plk1 activity and induces abscission failure and multinucleation, which were rescued upon expression of wild type Optn but not a phosphodeficient mutant (S177A) that cannot translocate into the nucleus during mitosis. Overall, these results highlight an important role of Optn in the spatial and temporal coordination of Plk1 activity, which was never been demonstrated before.

### REFERENCES

Albagha, O.M., Visconti, M.R., Alonso, N., Langston, A.L., Cundy, T., Dargie, R., Dunlop, M.G., Fraser, W.D., Hooper, M.J., Isaia, G., et al. (2010). Genome-wide association study identifies variants at CSF1, OPTN and TNFRSF11A as genetic risk factors for Paget's disease of bone. Nat Genet 42, 520-524.
Archambault, V., and Glover, D.M. (2009). Polo-like kinases: conservation and divergence in their functions and regulation. Nat Rev Mol Cell Biol 10, 265-275.
Arden, S.D., Puri, C., Au, J.S., Kendrick-Jones, J., and Buss, F. (2007). Myosin VI is required for targeted membrane transport during cytokinesis. Mol Biol Cell 18, 4750-4761.
Bastos, R.N., and Barr, F.A. (2010). Plk1 negatively regulates Cep55 recruitment to the midbody to ensure orderly abscission. J Cell Biol 191, 751-760.
Brennan, I.M., Peters, U., Kapoor, T.M., and Straight, A.F. (2007). Polo-like kinase controls vertebrate spindle elongation and cytokinesis. PLoS One 2, e409.
Burckstummer, T., Bennett, K.L., Preradovic, A., Schutze, G., Hantschel, O., Superti-Furga, G., and Bauch, A. (2006). An efficient tandem affinity purification procedure for interaction proteomics in mammalian cells. Nat Methods 3, 1013-1019.
Campbell, K.S., Cooper, S., Dessing, M., Yates, S., and Buder, A. (1998). Interaction of p59fyn kinase with the dynein light chain, Tctex-1, and colocalization during cytokinesis. J Immunol 161, 1728-1737.
Chalasani, M.L., Swarup, G., and Balasubramanian, D. (2009). Optineurin and its mutants: molecules associated with some forms of glaucoma. Ophthalmic Res 42, 176-184.
Chung, P.Y., Beyens, G., Boonen, S., Papapoulos, S., Geusens, P., Karperien, M., Vanhoenacker, F., Verbruggen, L., Fransen, E., Van Offel, J., et al. (2010). The majority of the genetic risk for Paget's disease of bone is explained by genetic variants close to the CSF1, OPTN, TM7SF4, and TNFRSF11A genes. Hum Genet 128, 615-626.
De Marco, N., Buono, M., Troise, F., and Diez-Roux, G. (2006). Optineurin increases cell survival and translocates to the nucleus in a Rab8-dependent manner upon an apoptotic stimulus. J Biol Chem 281, 16147-16156.
Delaval, B., Ferrand, A., Conte, N., Larroque, C., Hernandez-Verdun, D., Prigent, C., and Birnbaum, D. (2004). Aurora B -TACC1 protein complex in cytokinesis. Oncogene 23, 4516-4522.
Elia, A.E., Rellos, P., Haire, L.F., Chao, J.W., Ivins, F.J., Hoepker, K., Mohammad, D., Cantley, L.C., Smerdon, S.J., and Yaffe, M.B. (2003). The molecular basis for phosphodependent substrate targeting and regulation of Plks by the Polo-box domain. Cell 115, 83-95.
Glotzer, M. (2005). The molecular requirements for cytokinesis. Science 307, 1735-1739.
Hattula, K., and Peranen, J. (2000). FIP-2, a coiled-coil protein, links Huntingtin to Rab8 and modulates cellular morphogenesis. Curr Biol 10, 1603-1606.
Israel, A. (2006). NF-kappaB activation: Nondegradative ubiquitination implicates NEMO. Trends Immunol 27, 395-397.
Karki, S., LaMonte, B., and Holzbaur, E.L. (1998). Characterization of the p22 subunit of dynactin reveals the localization of cytoplasmic dynein and dynactin to the midbody of dividing cells. J Cell Biol 142, 1023-1034.
Kouranti, I., Sachse, M., Arouche, N., Goud, B., and Echard, A. (2006). Rab35 regulates an endocytic recycling pathway essential for the terminal steps of cytokinesis. Curr Biol 16, 1719-1725.
Li, J., Abraham, S., Cheng, L., Witzmann, F.A., Koch, M., Xie, J., Rahman, M., and Mohammed, S.I. (2008). Proteomic-based approach for biomarkers discovery in early detection of invasive urothelial carcinoma. Proteomics Clin Appl 2, 78-89.
Litvak, V., Argov, R., Dahan, N., Ramachandran, S., Amarilio, R., Shainskaya, A., and Lev, S. (2004). Mitotic phosphorylation of the peripheral Golgi protein Nir2 by Cdk1 provides a docking mechanism for Plk1 and affects cytokinesis completion. Mol Cell 14, 319-330.
Liu, X., and Erikson, R.L. (2003). Polo-like kinase (Plk)1 depletion induces apoptosis in cancer cells. Proc Natl Acad Sci U S A 100, 5789-5794.
Lobry, C., Lopez, T., Israel, A., and Weil, R. (2007). Negative feedback loop in T cell activation through IkappaB kinase-induced phosphorylation and degradation of Bcl10. Proc Natl Acad Sci U S A 104, 908-913.
Macurek, L., Lindqvist, A., Lim, D., Lampson, M.A., Klompmaker, R., Freire, R., Clouin, C., Taylor, S.S., Yaffe, M.B., and Medema, R.H. (2008). Polo-like kinase-1 is activated by aurora A to promote checkpoint recovery. Nature 455, 119-123.
Maruyama, H., Morino, H., Ito, H., Izumi, Y., Kato, H., Watanabe, Y., Kinoshita, Y., Kamada, M., Nodera, H., Suzuki, H., et al. (2010). Mutations of optineurin in amyotrophic lateral sclerosis. Nature 465, 223-226.
Nagabhushana, A., Chalasani, M.L., Jain, N., Radha, V., Rangaraj, N., Balasubramanian, D., and Swarup, G. (2010). Regulation of endocytic trafficking of transferrin receptor by optineurin and its impairment by a glaucoma-associated mutant. BMC Cell Biol 11, 4.
Nakajima, H., Toyoshima-Morimoto, F., Taniguchi, E., and Nishida, E. (2003). Identification of a consensus motif for Plk (Polo-like kinase) phosphorylation reveals Myt1 as a Plk1 substrate. J Biol Chem 278, 25277-25280.
Petronczki, M., Glotzer, M., Kraut, N., and Peters, J.M. (2007). Polo-like kinase 1 triggers the initiation of cytokinesis in human cells by promoting recruitment of the RhoGEF Ect2 to the central spindle. Dev Cell 12, 713-725.
Petronczki, M., Lenart, P., and Peters, J.M. (2008). Polo on the Rise-from Mitotic Entry to Cytokinesis with Plk1. Dev Cell 14, 646-659.
Rezaie, T., Child, A., Hitchings, R., Brice, G., Miller, L., Coca-Prados, M., Heon, E., Krupin, T., Ritch, R., Kreutzer, D., et al. (2002). Adult-onset primary open-angle glaucoma caused by mutations in optineurin. Science 295, 1077-1079.
Sahlender, D.A., Roberts, R.C., Arden, S.D., Spudich, G., Taylor, M.J., Luzio, J.P., Kendrick-Jones, J., and Buss, F. (2005). Optineurin links myosin VI to the Golgi complex and is involved in Golgi organization and exocytosis. J Cell Biol 169, 285-295.
Salaun, P., Rannou, Y., and Prigent, C. (2008). Cdk1, Plks, Auroras, and Neks: the mitotic bodyguards. Adv Exp Med Biol 617, 41-56.
Santamaria, A., Neef, R., Eberspacher, U., Eis, K., Husemann, M., Mumberg, D., Prechtl, S., Schulze, V., Siemeister, G., Wortmann, L., et al. (2007). Use of the novel Plk1 inhibitor ZK-thiazolidinone to elucidate functions of Plk1 in early and late stages of mitosis. Mol Biol Cell 18, 4024-4036.
Satyanarayana, A., and Kaldis, P. (2009). Mammalian cell-cycle regulation: several Cdks, numerous cyclins and diverse compensatory mechanisms. Oncogene 28, 2925-2939.
Schwamborn, K., Weil, R., Courtois, G., Whiteside, S.T., and Israel, A. (2000). Phorbol esters and cytokines regulate the expression of the NEMO-related protein, a molecule involved in a NF-kappa B-independent pathway. J Biol Chem 275, 22780-22789.
Seki, A., Coppinger, J.A., Jang, C.Y., Yates, J.R., and Fang, G. (2008). Bora and the kinase Aurora a cooperatively activate the kinase Plk1 and control mitotic entry. Science 320, 1655-1658.
Sumara, I., Gimenez-Abian, J.F., Gerlich, D., Hirota, T., Kraft, C., de la Torre, C., Ellenberg, J., and Peters, J.M. (2004). Roles of polo-like kinase 1 in the assembly of functional mitotic spindles. Curr Biol 14, 1712-1722.
Vassilev, L.T., Tovar, C., Chen, S., Knezevic, D., Zhao, X., Sun, H., Heimbrook, D.C., and Chen, L. (2006). Selective small-molecule inhibitor reveals critical mitotic functions of human CDK1. Proc Natl Acad Sci U S A 103, 10660-10665.
Vetterkind, S., Lee, E., Sundberg, E., Poythress, R.H., Tao, T.C., Preuss, U., and Morgan, K.G. (2010). Par-4: a new activator of myosin phosphatase. Mol Biol Cell 21, 1214-1224.
Wild, P., Farhan, H., McEwan, D.G., Wagner, S., Rogov, V.V., Brady, N.R., Richter, B., Korac, J., Waidmann, O., Choudhary, C., et al. (2011). Phosphorylation of the Autophagy Receptor Optineurin Restricts Salmonella Growth. Science 333, 228-233
Yamashiro, S., Yamakita, Y., Totsukawa, G., Goto, H., Kaibuchi, K., Ito, M., Hartshorne, D.J., and Matsumura, F. (2008). Myosin phosphatase-targeting subunit 1 regulates mitosis by antagonizing polo-like kinase 1. Dev Cell 14, 787-797.
Burckstummer, T., Bennett, K.L., Preradovic, A., Schutze, G., Hantschel, O., Superti-Furga, G., and Bauch, A. (2006). An efficient tandem affinity purification procedure for interaction proteomics in mammalian cells. Nat Methods 3, 1013-1019.
Izumi, T., and Maller, J.L. (1993). Elimination of cdc2 phosphorylation sites in the cdc25 phosphatase blocks initiation of M-phase. Mol Biol Cell 4, 1337-1350.
Journo, C., Filipe, J., About, F., Chevalier, S.A., Afonso, P.V., Brady, J.N., Flynn, D., Tangy, F., Israel, A., Vidalain, P.O., et al. (2009). NRP/Optineurin Cooperates with TAX1 BP1 to potentiate the activation of NF-kappaB by human T-lymphotropic virus type 1 tax protein. PLoS Pathog 5, e1000521.
Kumagai, A., and Dunphy, W.G. (1992). Regulation of the cdc25 protein during the cell cycle in Xenopus extracts. Cell 70, 139-151.
Yamashiro, S., Yamakita, Y., Totsukawa, G., Goto, H., Kaibuchi, K., Ito, M., Hartshorne, D.J., and Matsumura, F. (2008). Myosin phosphatase-targeting subunit 1 regulates mitosis by antagonizing polo-like kinase 1. Dev Cell 14, 787-797.

## Claims

1. A polypeptide consisting of a polypeptidic sequence disclosed in SEQ ID N°2 or having a polypeptidic sequence encompassing the polypeptidic sequence disclosed in SEQ ID N°2 which represents the portion of the wild-type human Optineurin protein sequence from its amino-acid residue 131 to its amino-acid residue 297, or having a polypeptidic sequence derived from the polypeptidic sequence disclosed in SEQ ID N°2 to the exclusion of the wild-type Optineurin protein.

2. The polypeptide of Claim 1, whose polypeptidic sequence comprises a Plk1 phosphorylation consensus sequence D/E-X-S-Φ-X-D/E, X being any amino acid residue, Φ being a hydrophobic amino acid residue, especially a Plk1 phosphorylation consensus sequence comprising amino-acid residue at position 177 by reference to the Optineurin protein sequence SEQ ID N°1.

3. The polypeptide of any one of Claims 1 or 2, whose polypeptidic sequence comprises any one of SEQ ID N°4 to SEQ ID N°8.

4. The polypeptide of any one of Claims 1 to 3, which retains the capacity of the wild-type human Optineurin protein to bind MYPT1 protein, when said MYPT1 protein is engaged in a Myosin-Phosphatase (MP) complex, and/or retains the capacity of the wild-type human Optineurin protein to bind Rab8 protein, when the latter are associated with the Golgi apparatus of a cell.

5. The polypeptide of any one of Claims 1 to 4, whose polypeptidic sequence comprises a Leucine Zipper (LZ) domain, especially a Leucine Zipper (LZ) domain located upstream from its Plk1 phosphorylation consensus sequence, in particular located upstream of position 177 by reference to the Optineurin protein sequence SEQ ID N°1.

6. The polypeptide of any one of Claims 1 to 5, whose polypeptidic sequence consists of any one of the sequences selected amongst: the polypeptidic sequence from residue 131 to 180 of SEQ ID NO:1, from residue 141 to residue 180 of SEQ ID NO:1, or from residue 131 to residue 209 of SEQ ID NO:1, or from residue 141 to residue 209 of SEQ ID NO:1, or from residue 131 to residue 219 of SEQ ID NO:1, or from residue 141 to residue 219 of SEQ ID NO:1.

7. The polypeptide of any one of Claims 2 to 6, whose polypeptidic sequence:
iii. is mutated in the Plk1 phosphorylation sequence in a way that it does not enable phosphorylation of said polypeptide, especially by Plk1, in particular is mutated at position 177 by reference to the Optineurin protein sequence SEQ ID N°1 so as to prevent phosphorylation of the amino-acid residue at said position 177, in particular has an Alanine residue at said position 177, and,
iv. optionally does not have the capacity of the wild-type human Optineurin protein to bind MYPT1 protein, when said MYPT1 protein is engaged in a Myosin-Phosphatase (MP) complex.

8. The polypeptide of Claim 7, whose polypeptidic sequence is mutated in a portion encompassing a Leucine Zipper (LZ) domain located upstream from said polypeptide mutated Plk1 phosphorylation consensus sequence, so as to disrupt the function of said Leucine Zipper (LZ) domain, or is devoid of such a Leucine Zipper (LZ) domain.

9. The polypeptide according to any one of Claims 1 to 8, which has the amino-acid sequence SEQ ID N°2 or SEQ ID N°3 or SEQ ID N°10.

10. A nucleic acid molecule encoding a polypeptide according to any one of Claims 1 to 9.

11. A vector, which is a cloning vector or an expression vector, especially a plasmid, comprising a nucleic acid molecule according to Claim 10.

12. A method for producing a polypeptide according to any one of Claims 1 to 9 comprising the transfection of a cell with a vector according to Claim 10, the culture of said cell and the recovery of said polypeptide.

13. A composition comprising a polypeptide according to any one of Claims 1 to 9, in particular a pharmaceutical composition further comprising pharmaceutically acceptable excipient(s), especially carrier(s) and/or adjuvant(s).

14. A polypeptide according to any one of Claims 1 to 9, or a composition according to Claim 13, for use in a method of therapy practised on a human or animal body, in particular for use in treating a disease selected from the following group: neoplasic disease(s), including cancer(s), Optineurin-mutation(s) linked diseases, primary open-angle glaucoma, juvenile open-angle glaucoma, amyotrophic lateral sclerosis, Huntington disease.

15. Polypeptide for use either to enhance or to counteract negative regulation of Plk1 activity by Optineurin in a dividing cell, thereby impairing or therapeutically interfering with the cascade of events involved in said cell when undergoing mitosis, wherein said polypeptide binds in said cell to at least one cellular partner for Optineurin selected amongst Rab8 protein and MYPT1 protein, including when said MYPT1 protein is engaged in a Myosin-Phosphatase (MP) complex, and thereby modulates the subcellular distribution of the endogenous Optineurin protein between the cytoplasm and the nucleus in said cell.

16. Polypeptide according to Claim 15, for use as a medicament to prevent cell mitosis completion, in particular in transformed cells or infected cells.

17. Polypeptide according to any one of Claims 15 or 16, for preventing or treating a disease involving mitosis deregulation(s) or alteration(s), such as a neoplasic disease or cancer, in an animal or human body..

18. Nucleic acid molecule, vector, method or composition according to any one of Claims 10 to 13, for use according to any one of Claims 15 to 17.

19. *In vitro* screening method for identifying compound(s) capable of specifically activating the phosphorylation of endogenous Optineurin and/or enhancing endogenous Optineurin translocation into the nucleus of a cell, thereby negatively regulating Plk1 activity in a cell, comprising the steps of:
i. contacting a cell with an antibody specifically recognizing either the Optineurin protein or an Optineurin-mimicking polypeptide with a Plk1 phosphorylation consensus sequence D/E-X-S-Φ-X-D/E, X being any amino acid residue; Φ being a hydrophobic amino acid residue, whose Serine amino-acid residue is phosphorylated, especially an Optineurin-mimicking polypeptide that is phosphorylated at position 177 by reference to the Optineurin protein sequence SEQ ID N°1,
ii. contacting said cell with compound(s) to assay, wherein steps i. and ii. can be inverted,
iii. visualizing the change(s) in properties of the cell contacted in steps i. and ii., and,
iv. optionally, recording or quantifying the change(s) in properties of the cell contacted in steps i) and ii), and,
v. optionally, detecting the activity of Plk1, in particular detecting the phosphorylation on Threonine 210 residue of Plk1.
